(19) Europäisches Patentamt
European Patent Office
Office européen des brevets

(11) EP 3 508 203 A1

(12) EUROPEAN PATENT APPLICATION
published in accordance with Art. 153(4) EPC

(43) Date of publication:
10.07.2019 Bulletin 2019/28

(21) Application number: 17846617.3

(22) Date of filing: 30.08.2017

(51) Int Cl.:
*A61K 31/496* (2006.01) *A61K 9/10* (2006.01)
*A61K 47/02* (2006.01) *A61K 47/10* (2017.01)
*A61K 47/14* (2017.01) *A61K 47/26* (2006.01)
*A61K 47/32* (2006.01) *A61K 47/36* (2006.01)
*A61K 47/38* (2006.01)

(86) International application number:
PCT/JP2017/031275

(87) International publication number:
WO 2018/043613 (08.03.2018 Gazette 2018/10)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA MD

(30) Priority: 31.08.2016 JP 2016169596

(71) Applicant: Sumitomo Dainippon Pharma Co., Ltd.
Osaka-shi
Osaka 541-8524 (JP)

(72) Inventors:
- TSUZUKU, Takuma
Suita-shi
Osaka 564-0053 (JP)
- ONITA, Maiko
Suita-shi
Osaka 564-0053 (JP)

(74) Representative: Vossius & Partner
Patentanwälte Rechtsanwälte mbB
Siebertstrasse 3
81675 München (DE)

# (54) AQUEOUS SUSPENSION PREPARATION

(57) The present invention provides an aqueous suspension preparation comprising (3aR,4S,7R,7aS)-2-{(1R,2R)-2-[4-(1,2-benzisothiazol-3-yl)piperazin-1-ylmethyl] cyclohexylmethyl}hexahydro-4, 7-methano-2H-isoindole-1,3-dione (Compound 1), a pharmaceutically acceptable acid addition salt thereof, or a mixture thereof as an active ingredient, which has the following feature (I) or (II):
(I) the circularity of the suspended particle is about 0.960 or less when the preparation comprises a thickening agent,
(II) the circularity of the suspended particle is about 0.945 or less when the preparation comprises no thickening agent.

EP 3 508 203 A1

**Description**

TECHNICAL FIELD

The present invention relates to an aqueous suspension preparation. In detail, the present invention relates to an aqueous suspension preparation comprising (3aR,4S,7R,7aS)-2-{(1R,2R)-2-[4-(1,2-benzisothiazol-3-yl)piperazin-1-ylmethyl]cyclohexylmethyl}hexahydro-4,7-methano-2H-isoindol-1,3-dione (hereinafter also referred to as "Compound 1"), a pharmaceutically acceptable acid addition salt thereof, or a mixture thereof (hereinafter also referred to as "Compound 1 or salt thereof") which is suspended in a solvent including water.

BACKGROUND ART

It is well known that (3aR,4S,7R,7aS)-2-{(1R,2R)-2-[4-(1,2-benzisothiazol-3-yl)piperazin-1-ylmethyl]cyclohexylmethyl}hexahydro-4,7-methano-2H-isoindol-1,3-dione (Compound 1) is a compound used as an atypical antipsychotic agent, and the compound is useful in the treatment of a psychiatric disease such as schizophrenia, bipolar disorder, and depression (e.g. Patent Documents 1 and 2).

In general, an oral solid preparation such as tablet and capsule has been widely used in the treatment of various diseases because it is an easily administrable dosage form which can be taken at home. On the other hand, there are some situations which cannot be sufficiently treated by only the use of an oral solid preparation, in case of, for example, the administration to a patient suffering from acute schizophrenia, the administration to a patient in particular need of adjusting a dosage like children and senior people, the administration to a patient who refuses to take a drug, and the administration to a patient who has difficulty in swallowing, because the psychiatric disease such as schizophrenia causes various symptoms for each patient. Thus, some attempts to use an oral solution and an injection which are more suitable for the administration to such patients have been done. In order to prepare such oral solution or such injection, it is necessary to dissolve or suspend a drug into a solvent.

As an oral solution of Compound 1, a solution preparation comprising N-[4-[4-(1,2-benzisothiazol-3-yl)-1-piperazinyl]-(2R,3R)-2,3-tetramethylene-butyl]-(1'R,2'S,3'R,4'S)-2,3-bicyclo[2,2,1]heptanedicarboxyimide hydrochloride which is Compound 1 hydrochloride as an active ingredient and at least one substance selected from benzyl alcohol, N,N-dimethylacetamide, lactic acid or propylene glycol has ever been reported (Patent Document 3).

Also, Patent Document 4 discloses a composition comprising N-[4-[4-(1,2-benzisothiazol-3-yl)-1-piperazinyl]-(2R,3R)-2,3-tetramethylene-butyl]-(1'R,2'S,3'R,4'S)-2,3-bicyclo[2,2,1]heptanedicarboxyimide which is Compound 1, or a pharmaceutically acceptable acid addition salt thereof, and more specifically a sustained-release preparation for injection which maintains an effective blood level of the compound. However, Patent Document 4 discloses no information on the preparation usability such as the precipitation of a drug in the form of suspended particle and the redispersibility of the preparation after the precipitation.

When an aqueous suspension preparation is allowed to stand for a long time, a drug in the form of suspended particle is precipitated and solidified. Thus, it has been required to develop an aqueous suspension preparation in which the suspended particle is difficult to be precipitated or an aqueous suspension preparation with the feature in which the precipitated suspended particle can easily return to the initial suspended state.

PRIOR ART DOCUMENTS

PATENT DOCUMENTS

Patent Document 1: JP 2800953 B (US 5532372 A)
Patent Document 2: WO 2005/009999
Patent Document 3: WO 2006/134864
Patent Document 4: WO 2012/053654

SUMMARY OF INVENTION

(PROBLEM TO BE SOLVED BY THE INVENTION)

When an aqueous suspension preparation is actually used in the clinical practice, the preparation is basically shaken by a patient or a medical staff prior to administration. In case that the preparation has poor redispersibility, the shaking may cause the different dosage depending on each person. Thus, when an aqueous suspension preparation

is developed, it is necessary to make some pharmaceutical attempts to enhance the preparation usability such as redispersibility so as to reduce the variation in the dosage depending on each user or each use.

The present invention has been studied considering the above situation, and an object of the present invention is to provide an aqueous suspension preparation comprising Compound 1 or salt thereof as an active ingredient, wherein the redispersibility of the preparation is good, which has the feature in which the suspended particle can easily return to the initial dispersed state by a simple operation when the particle is precipitated by standing for a long time.

(MEANS FOR SOLVING THE PROBLEMS)

The present inventors have extensively studied to reach the above object, and then have found that a preparation wherein the circularity of the suspended particle is below a certain value unexpectedly shows good redispersibility in analyzing the particle image of each suspended particle with a flow particle image analyzer, and thus there is a definite relation between the result of the redispersibility and the particle image. In particular, the present inventors have analyzed the circularity of each suspended particle with a flow particle image analyzer, and then have found that the aqueous suspension preparation comprising no thickening agent, wherein the circularity of the suspended particle is about 0.945 or less, or the aqueous suspension preparation comprising a thickening agent, wherein the circularity of the suspended particle is about 0.960 or less shows good redispersibility. Based upon the new findings, the present invention has been completed.

In addition, when a solution of Compound 1 or salt thereof is orally administered, it produces strong bitter taste. Whereas, the aqueous suspension preparation of the present invention is expected to produce no or little bitter taste because the amount of Compound 1 or salt thereof dissolved therein is small, and thus the preparation is seemed to have a good administration feeling.

The present invention provides inventions described below.

[Item 1] An aqueous suspension preparation comprising (3aR,4S,7R,7aS)-2-{(1R,2R)-2-[4-(1,2-benzisothiazol-3-yl)piperazin-1-ylmethyl]cyclohexylmethyl}hexahydro-4,7-methano-2H-isoindole-1,3-dione (Compound 1), a pharmaceutically acceptable acid addition salt thereof, or a mixture thereof as an active ingredient, which has the following feature (I) or (II):

(I) the circularity of the suspended particle is about 0.960 or less when the preparation comprises a thickening agent,
(II) the circularity of the suspended particle is about 0.945 or less when the preparation comprises no thickening agent.

[Item 2] An aqueous suspension preparation comprising (3aR,4S,7R,7aS)-2-{(1R,2R)-2-[4-(1,2-benzisothiazol-3-yl)piperazin-1-ylmethyl]cyclohexylmethyl}hexahydro-4,7-methano-2H-isoindole-1,3-dione (Compound 1), a pharmaceutically acceptable acid addition salt thereof, or a mixture thereof wherein the circularity of the suspended particle is about 0.945 or less.
[Item 3] The preparation according to the item 1 or 2, wherein the median diameter of the suspended particle of Compound 1, a pharmaceutically acceptable acid addition salt thereof, or a mixture thereof is about 0.1 - about 30 $\mu$m.
[Item 4] The preparation according to any one of the items 1 to 3 wherein the Rsp value of Compound 1, a pharmaceutically acceptable acid addition salt thereof, or a mixture thereof suspended in the preparation which is measured with pulse NMR is about 0.25 or more, wherein said measured concentration is diluted to 40 mg/mL (in terms of Compound 1 hydrochloride).
[Item 5] The preparation according to any one of the items 1 to 4, wherein the content of Compound 1, a pharmaceutically acceptable acid addition salt thereof, or a mixture thereof is about 0.1 - about 400 mg/mL in terms of Compound 1 hydrochloride.
[Item 6] The preparation according to any one of the items 1 to 5 which further comprises a dispersant.
[Item 7] The preparation according to the item 6, wherein the dispersant is at least one ingredient selected from the group consisting of cellulose derivatives, sucrose fatty acid esters and polyvinyl alcohol.
[Item 8] The preparation according to the item 6, wherein the dispersant is at least one ingredient selected from the group consisting of cellulose derivatives and sucrose fatty acid esters.
[Item 9] The preparation according to any one of the items 6 to 8, wherein the content of the dispersant is about 0.1 - about 20 mg/mL.
[Item 10] The preparation according to any one of the items 1 to 9 which comprises a thickening agent.
[Item 11] The preparation according to the item 10, wherein the thickening agent is at least one ingredient selected from polysaccharides.
[Item 12] The preparation according to the item 10 or 11, wherein the content of the thickening agent is about 0.5 -

about 20 mg/mL.

[Item 13] The preparation according to any one of the items 1 to 12 which further comprises a buffering agent.

[Item 14] The preparation according to the item 13, wherein the buffering agent is at least one ingredient selected from the group consisting of sodium salt, potassium salt and hydrate thereof.

[Item 15] The preparation according to the item 13 or 14, wherein the buffering agent is contained in an amount of about 0.01 - about 15 mg relative to 1 mg of Compound 1, a pharmaceutically acceptable acid addition salt thereof, or a mixture thereof in terms of Compound 1 hydrochloride.

[Item 16] The preparation according to any one of the items 1 to 15 which further comprises a preservative.

[Item 17] The preparation according to the item 16, wherein the preservative is at least one ingredient selected from benzoic acid derivatives.

[Item 18] The preparation according to the item 16 or 17, wherein the content of the preservative is about 0.1 - about 10 mg/mL.

[Item 19] The preparation according to any one of the items 1 to 18 which further comprises a stabilizing agent.

[Item 20] The preparation according to the item 19, wherein the stabilizing agent is at least one ingredient selected from polyalcohols.

[Item 21] The preparation according to the item 19 or 20, wherein the content of the stabilizing agent is about 1 - about 500 mg/mL.

[Item 22] The preparation according to any one of the items 1 to 21 wherein the circularity of the suspended particle is about 0.960 or less, comprising the following (1)-(3):

(1) Compound 1, a pharmaceutically acceptable acid addition salt thereof, or a mixture thereof in a content of about 0.1 - about 400 mg/mL in terms of Compound 1 hydrochloride,
(2) a dispersant in a content of about 0.1 - about 20 mg/mL, which is at least one ingredient selected from the group consisting of cellulose derivatives and sucrose fatty acid esters, and
(3) a thickening agent in a content of about 0.5 - about 20 mg/mL, which is at least one ingredient selected from polysaccharides.

[Item 23] The preparation according to the item 22, wherein

(1) the content of Compound 1, a pharmaceutically acceptable acid addition salt thereof, or a mixture thereof is about 10 - about 120 mg/mL in terms of Compound 1 hydrochloride,
(2) the content of the dispersant is about 0.2 - about 20 mg/mL, and
(3) the content of the thickening agent is about 2 - about 10 mg/mL.

[Item 24] The preparation according to any one of the items 1 to 21 comprising the following (1)-(4):

(1) Compound 1, a pharmaceutically acceptable acid addition salt thereof, or a mixture thereof in a content of about 0.1 - about 400 mg/mL in terms of Compound 1 hydrochloride,
(2) a dispersant in a content of about 0.1 - about 20 mg/mL, which is at least one ingredient selected from the group consisting of cellulose derivatives and sucrose fatty acid esters,
(3) a buffering agent in a content of about 0.01 - about 15 mg relative to 1 mg of Compound 1, a pharmaceutically acceptable acid addition salt thereof, or a mixture thereof in terms of Compound 1 hydrochloride, which is at least one ingredient selected from the group consisting of sodium salt, potassium salt, and hydrate thereof, and
(4) a preservative in a content of about 0.1 - about 10 mg/mL, which is at least one ingredient selected from benzoic acid derivatives.

[Item 25] The preparation according to the item 24, wherein

(1) the content of Compound 1, a pharmaceutically acceptable acid addition salt thereof, or a mixture thereof is about 10 - about 120 mg/mL in terms of Compound 1 hydrochloride,
(2) the content of the dispersant is about 0.2 - about 20 mg/mL,
(3) the content of the buffering agent is about 0.125 - about 0.85 mg relative to 1 mg of Compound 1, a pharmaceutically acceptable acid addition salt thereof, or a mixture thereof in terms of Compound 1 hydrochloride, and
(4) the content of the preservative is about 0.5 - about 2.5 mg/mL.

[Item 26] The preparation according to any one of the items 1 to 21 wherein the circularity of the suspended particle is about 0.960 or less, comprising the following (1)-(6):

(1) Compound 1, a pharmaceutically acceptable acid addition salt thereof, or a mixture thereof in a content of about 0.1 - about 400 mg/mL in terms of Compound 1 hydrochloride,

(2) a dispersant in a content of about 0.1 - about 20 mg/mL, which is at least one ingredient selected from the group consisting of cellulose derivatives and sucrose fatty acid esters,

(3) a thickening agent in a content of about 0.5 - about 20 mg/mL, which is at least one ingredient selected from polysaccharides,

(4) a buffering agent in a content of about 0.01 - about 15 mg relative to 1 mg of Compound 1, a pharmaceutically acceptable acid addition salt thereof, or a mixture thereof in terms of Compound 1 hydrochloride, which is at least one ingredient selected from sodium salt, potassium salt, and hydrate thereof,

(5) a preservative in a content of about 0.1 - about 10 mg/mL, which is at least one ingredient selected from benzoic acid derivatives, and

(6) a stabilizing agent in a content of about 1 - about 500 mg/mL, which is at least one ingredient selected from polyalcohols.

[Item 27] The preparation according to the item 26, wherein

(1) the content of Compound 1, a pharmaceutically acceptable acid addition salt thereof, or a mixture thereof is about 10 - about 120 mg/mL in terms of Compound 1 hydrochloride,

(2) the content of the dispersant is about 0.2 - about 20 mg/mL,

(3) the content of the thickening agent is about 2 - about 10 mg/mL,

(4) the content of the buffering agent is about 0.125 - about 0.85 mg relative to 1 mg of Compound 1, a pharmaceutically acceptable acid addition salt thereof, or a mixture thereof in terms of Compound 1 hydrochloride,

(5) the content of the preservative is about 0.5 - about 2.5 mg/mL, and

(6) the content of the stabilizing agent is about 50 - about 150 mg/mL.

[Item 28] The preparation according to any one of the items 1 to 27 which comprises Compound 1 hydrochloride as Compound 1, a pharmaceutically acceptable acid addition salt thereof, or a mixture thereof.

[Item 29] The preparation according to any one of the items 6 to 28, wherein the dispersant is at least one ingredient selected from the group consisting of hypromellose, hydroxypropylcellulose, sucrose laurate and methylcellulose.

[Item 30] The preparation according to the item 29 which comprises hypromellose as the dispersant.

[Item 31] The preparation according to any one of the items 10 to 23 or 26 to 30, wherein the thickening agent is at least one ingredient selected from the group consisting of xanthan gum, guar gum, carrageenan, and sodium alginate.

[Item 32] The preparation according to the item 31, wherein the thickening agent is at least one ingredient selected from the group consisting of xanthan gum, guar gum, and sodium alginate.

[Item 33] The preparation according to the item 31 which comprises xanthan gum as the thickening agent.

[Item 34] The preparation according to the item 31 which comprises sodium alginate as the thickening agent.

[Item 35] The preparation according to any one of the items 13 to 21 or 24 to 34, wherein the buffering agent is at least one ingredient selected from the group consisting of dipotassium phosphate, monopotassium phosphate, disodium phosphate, monosodium phosphate, potassium chloride, sodium chloride, sodium citrate, and hydrate thereof.

[Item 36] The preparation according to the item 35, wherein the buffering agent is at least one ingredient selected from the group consisting of dipotassium phosphate, monopotassium phosphate, disodium phosphate, monosodium phosphate, sodium chloride, and hydrate thereof.

[Item 37] The preparation according to item 35, wherein the buffering agent is at least one ingredient selected from the group consisting of dipotassium phosphate, monopotassium phosphate, sodium chloride, and hydrate thereof.

[Item 38] The preparation according to the items 16 to 21 or 24 to 37, wherein the preservative is at least one ingredient selected from the group consisting of methyl parahydroxybenzoate, ethyl parahydroxybenzoate, propyl parahydroxybenzoate, butyl parahydroxybenzoate, and sodium benzoate.

[Item 39] The preparation according to the item 38, wherein the preservative is at least one ingredient selected from the group consisting of methyl parahydroxybenzoate, propyl parahydroxybenzoate, and sodium benzoate.

[Item 40] The preparation according to the item 38 which comprises methyl parahydroxybenzoate as the preservative.

[Item 41] The preparation according to any one of the items 19 to 21 or 26 to 40, wherein the stabilizing agent is at least one ingredient selected from the group consisting of propylene glycol, glycerin, polyethyleneglycol, and ethanol.

[Item 42] The preparation according to the item 41 which comprises propylene glycol as the stabilizing agent.

[Item 43] The preparation according to any one of the items 1 to 42, wherein the content of Compound 1, a pharmaceutically acceptable acid addition salt thereof, or a mixture thereof is about 12 - about 100 mg/mL in terms of Compound 1 hydrochloride.

[Item 44] The preparation according to any one of the items 1 to 42, wherein the content of Compound 1, a pharmaceutically acceptable acid addition salt thereof, or a mixture thereof is about 25 - about 50 mg/mL in terms of Compound 1 hydrochloride.
[Item 45] The preparation according to any one of the items 1 to 44 which is an oral solution.
[Item 46] The preparation according to any one of the items 1 to 44 which is an injection.
[Item 47] A medicament for treatment and/or prophylaxis of a psychiatric disease, which comprises the preparation of any one of the items 1 to 46.
[Item 48] The medicament according to the item 47, wherein the psychiatric disease is schizophrenia, bipolar disorder, senile dementia, or depression.
[Item 49] A method for treatment and/or prophylaxis of a psychiatric disease, which comprises administering the preparation according to any one of the items 1 to 46 to a patient in need thereof.
[Item 50] The method according to the item 49, wherein the psychiatric disease is schizophrenia, bipolar disorder, senile dementia, or depression.
[Item 51] Use of the preparation according to any one of the items 1 to 46 for the manufacture of a medicament for treatment and/or prophylaxis of a psychiatric disease.
[Item 52] The use according to the item 51, wherein the psychiatric disease is schizophrenia, bipolar disorder, senile dementia, or depression.
[Item 53] The preparation of any one of the items 1 to 46 for use in the treatment and/or prophylaxis of a psychiatric disease.
[Item 54] The preparation according to the item 53, wherein the psychiatric disease is schizophrenia, bipolar disorder, senile dementia, or depression.
[Item 55] An aqueous suspension preparation comprising (3aR,4S,7R,7aS)-2-{(1R,2R)-2-[4-(1,2-benzoisothiazole-3-yl)piperazine-1-ylmethyl]cyclohexylmethyl}hexahydro-4,7-methano-2H-isoindole-1,3-dione (Compound 1), a pharmaceutically acceptable acid addition salt thereof, or a mixture thereof as an active ingredient, which has the following feature (I) or (II):

(I) the circularity of the suspended particle is about 0.960 or less when the preparation comprises a thickening agent, which is at least one ingredient selected from the group consisting of xanthan gum, guar gum, carrageenan, and sodium alginate,
(II) the circularity of the suspended particle is about 0.945 or less when the preparation comprises no thickening agent, which is selected from the group consisting of xanthan gum, guar gum, carrageenan, and sodium alginate.

The item 55 may be combined with any or all of the features of the items 3 to 54. That is, as with the items 3 to 54 which are dependent on the item 1, the items which are dependent on the item 55 are also included in the aspects of the present invention.

(EFFECTS OF THE INVENTION)

The present invention provides an aqueous suspension preparation of Compound 1 or salt thereof with an excellent redispersibility. In the aqueous suspension preparation of the present invention, the precipitate formed by standing for a long time can easily return to the initial homogeneous suspended state. Thus, the aqueous suspension preparation can reduce the variation in the dosage depending on each user or each use.

DETAILED DESCRIPTION OF THE PREFERRED EMBODIMENTS

Hereinafter, the preferred embodiments of the present invention are explained in detail. However, the present invention is not limited to the following embodiments.
The present invention discloses an aqueous suspension preparation of Compound 1 or salt thereof suitable for oral administration or administration by injection as a medicament for the treatment of a disease such as schizophrenia.
The term "mixture thereof" in "Compound 1, a pharmaceutically acceptable acid addition salt thereof, or a mixture thereof" as used herein encompasses (1) a mixture of Compound 1 (in the free form) and a pharmaceutically acceptable acid addition salt of Compound 1 (which may be one or two or more acid addition salts), (2) a mixture of two or more pharmaceutically acceptable acid addition salts of Compound 1.
The term "oral solution" as used herein means a solution for oral administration, and the term "injection" means a preparation for being administered intravenously, intramuscularly, subcutaneously, or intradermally.
The term "aqueous suspension preparation" as used herein comprising Compound 1 or salt thereof as an active ingredient means a preparation in which the suspended particle of Compound 1 or salt thereof is dispersed in a solvent including water as main solvent. The "aqueous suspension preparation" as used herein may comprise a polar solvent

in an amount which produces no negative effect (e.g. bitter taste, astringent taste, astringent) on administration feeling of the preparation besides water.

The term "polar solvent" as used herein means a solvent with polarity which can be homogeneously mixed with water.

The term "*the Rsp value of Compound 1, a pharmaceutically acceptable acid addition salt thereof, or a mixture thereof suspended in the preparation which is measured with pulse NMR, wherein said measured concentration is diluted to 40 mg/mL (in terms of Compound 1 hydrochloride)*" as used herein means the following things: in case that the content of Compound 1, a pharmaceutically acceptable acid addition salt thereof, or a mixture thereof in the preparation is higher than 40 mg/mL (in terms of Compound 1 hydrochloride), it means the Rsp value obtained by the measurement of the preparation with pulse NMR, whose concentration is adjusted to 40 mg/mL (in terms of Compound 1 hydrochloride) by diluting the preparation with *"aqueous phase obtained by excluding suspended particle from suspension preparation*"; and in case that the content of Compound 1, a pharmaceutically acceptable acid addition salt thereof, or a mixture thereof is 40 mg/mL (in terms of Compound 1 hydrochloride), it means the Rsp value obtained by the measurement of the preparation with pulse NMR without dilution. In case that the content of Compound 1, a pharmaceutically acceptable acid addition salt thereof, or a mixture thereof in the preparation is lower than 40 mg/mL (in terms of Compound 1 hydrochloride), it means the Rsp value obtained by the measurement of the concentrated preparation with pulse NMR, whose concentration is adjusted to 40 mg/mL (in terms of Compound 1 hydrochloride).

The term "good redispersibility" of the aqueous suspension preparation as used herein means the feature which can easily return to the initial homogeneous suspended state by a simple operation even if suspended particle in the aqueous suspension preparation is precipitated by standing for a long time.

The term "simple operation" as used herein means an operation such as inversion and shaking of the present preparation in a storage container, but is not limited thereto.

(I) Evaluation method of redispersibility

In the present invention, the redispersibility of a preparation can be determined, for example, by the following procedure: a test tube is filled with the aqueous suspension preparation and sealed, it is allowed to stand at 25°C or 40°C for a few months to make the suspended particle in the preparation spontaneously precipitated, and then the aspect after gently-inverting the test tube over about 3 seconds is checked about whether the precipitation of the particle is loosened or not, and whether the particle returns to the initial suspended state soon after the production or not. Specifically, when it was visually confirmed that the precipitation of the suspended particle in the test tube was loosened by one inversion and the suspended particle returned to the initial suspended state soon after the production, the redispersibility of the preparation was determined as being remarkably good (A). Also, when the suspended particle returned to the initial suspended state while the inversion was repeated five times, the redispersibility of the preparation was determined as being good (B). Whereas, when the suspended particle did not return to the initial suspended state by repeating the inversion five times, the redispersibility of the preparation was determined as being poor (C). Good redispersibility of the preparation denotes the state of (A) or (B).

The term "circularity" as used herein is one of the information on the shape of a suspended particle, and represents an index for representing the circular degree of a particle. That is, the circularity is a numeric index of not more than 1, which means that the particle is closer to a circular shape as the value approaches 1. Also, the circularity as used herein shows the average of the circularity of each particle in the suspended state. In general, the circularity of a particle is calculated from the image data of the particle. The circularity as used herein can be measured with flow particle image analyzer: FPIA3000 (Sysmex) and calculated according to the following formula.

Circularity = (Length around circle equal to area of projection image of particle)/(Length around projection image of particle)

(II) Measurement method of circularity

In the present invention, the circularity of the particle can be calculated, for example, by adding 5 mL of a solvent in which Compound 1 or salt thereof cannot be dissolved (e.g., purified water or neutral buffer solution) to 0.25 mL of the aqueous suspension preparation to prepare a measurement solution and by measuring the measurement solution with a flow particle image analyzer. Also, the circularity can be measured after appropriately-diluting the aqueous suspension preparation depending on the concentration of the preparation.

The term "median diameter" as used herein is one of the representative indexes for representing the particle size distribution of the suspended particle of Compound 1 or salt thereof in the aqueous suspension preparation, which means the middle diameter calculated from the volume standard. In general, the median diameter is measured with an analyzer such as laser diffraction particle size analyzer, dynamic light scattering particle size analyzer, and image processing particle size analyzer. The median diameter as used herein can be calculated from the particle size distribution measured with laser diffraction particle size analyzer: HELOS BR-MULTI (Sympatec).

(III) Measurement method of median diameter

In the present invention, the median diameter of the particle can be measured, for example, by adding 40 mL of a solvent in which Compound 1 or salt thereof cannot be dissolved (e.g., purified water or neutral buffer solution) to 0.01 mL of the aqueous suspension preparation to prepare a measurement solution and measuring the measurement solution with laser diffraction particle size analyzer. For example, when the median diameter is measured with HELOS BR-MULTI as the laser diffraction particle size analyzer, it is preferable to adjust the dilution rate of the measurement solution so that "Sample: Measurement Concentration" displayed during measurement becomes 5-10 %.

The "Rsp" as used herein means an inverse of the transverse relaxation time of protons whose movement is limited by Compound 1 or salt thereof in the suspended state in the aqueous suspension preparation, and represents an index for the amount of protons whose movement is limited or the degree of the limitation. The Rsp as used herein is the value calculated from the measurement of the preparation wherein the content of Compound 1 or salt thereof is adjusted to 40 mg/mL (in terms of Compound 1 hydrochloride) with pulse NMR. For example, the Rsp can be measured with pulse NMR particle boundary evaluation apparatus: Acorn area (xigo) and calculated according to the following formula.

Rsp = (*Inverse of transverse relaxation time of aqueous suspension preparation*)/(*Inverse of transverse relaxation time of aqueous phase obtained by excluding suspended particle from suspension preparation*) - 1

As for general calculation of Rsp, not only the inverse of transverse relaxation time as used herein but also the inverse of longitudinal relaxation time can be used in standard calculation of Rsp.

(IV) Measurement method of Rsp

The Rsp as used herein can be obtained by adjusting the content of Compound 1 or salt in the preparation to 40 mg/mL (in terms of Compound 1 hydrochloride) and by measuring the measurement solution as shown below.

A part of the aqueous suspension preparation is centrifuged with ultra-high speed centrifuge: himac CS150GX (HITACHI) at 100,000 rpm for 30 minutes to fully precipitate the suspended particle in the aqueous suspension preparation. When a visually clear supernatant is produced at this point, the supernatant is used as *"aqueous phase obtained by excluding suspended particle from suspension preparation"*. On the other hand, when the supernatant is not visually clear, the above centrifugation is repeated until the supernatant becomes clear. Using the measurement solution thus obtained from the *"aqueous phase obtained by excluding suspended particle from suspension preparation*", the transverse relaxation time can be measured with a pulse NMR particle boundary evaluation apparatus to obtain the value of the *"inverse of transverse relaxation time of aqueous phase obtained by excluding suspended particle from suspension preparation"* used for calculating Rsp. On the other hand, the value of the *"inverse of transverse relaxation time of aqueous suspension preparation"* can be obtained by preparing the measurement solution of the "aqueous suspension preparation" by repeating inverting and mixing until it visually becomes homogeneous, and then measuring the measurement solution with a pulse NMR particle boundary evaluation apparatus to obtain the transverse relaxation time thereof. The value of each transverse relaxation time thus obtained is applied to the above formula, and the Rsp of the aqueous suspension preparation can be calculated according to the formula.

The present inventors have extensively studied about the aqueous suspension preparations of the present invention, and then have found that good redispersibility can be unexpectedly achieved in the preparation when the Rsp values shows about 0.25 or more. The reason is considered as follows. The higher Rsp value means that the amount of protons whose movement is limited is high or the limitation degree is large. It means that there are many water molecules whose freedom is lost by the suspended particle, and thus it is thought that the water molecules whose

freedom is lost are bonded on the surface of the suspended particle. Thus, it is thought that the interaction between suspended particles is reduced and strong clumping and solidification hardly occurs because of many water molecules bonded on the surface of the suspended particle, and thus the redispersibility of the preparation is improved.

Compound 1 or salt thereof

Compound 1 of the present invention is (3aR,4S,7R,7aS)-2-{(1R,2R)-2-[4-(1,2-benzisothiazol-3-yl)piperazin-1-ylmethyl]cyclohexylmethyl}hexahydro-4,7-methano-2H-isoindole-1,3-dione, which is a compound of the following formula:

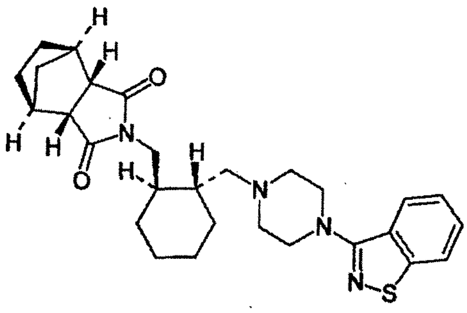

Compound 1 or salt thereof has a pharmacological effect of an antipsychotic agent. More specifically, Compound 1 or salt thereof and a pharmaceutical preparation comprising it are useful as a medicament for treating a psychiatric disease such as schizophrenia, bipolar disorder, and depression.

Examples of the pharmaceutically acceptable acid addition salt of Compound 1 include an organic acid addition salt and an inorganic acid addition salt. Examples of the organic acid addition salt include formate, acetate, lactate, adipate, citrate, tartrate, methanesulfonate, fumarate, and maleate, and examples of the inorganic acid addition salt include hydrochloride, sulfate, nitrate, and phosphate, but are not limited thereto. The pharmaceutically acceptable acid addition salt of Compound 1 is preferably hydrochloride. In addition, Compound 1 and a pharmaceutically acceptable acid addition salt thereof may be in the form of a solvate, a hydrate or a nonhydrate.

Compound 1 or salt thereof can be prepared according to, for example, the processes of Patent Documents 1 and 2 or similar processes thereto. The prepared Compound 1 or salt thereof may be milled according to a commonly-used method as appropriate.

The content of "Compound 1, a pharmaceutically acceptable acid addition salt thereof, or a mixture thereof (Compound 1 or salt thereof)" in the aqueous suspension preparation of the present invention may be changed depending on the amount of drug solution, but is basically about 0.1 - about 400 mg/mL in terms of Compound 1 hydrochloride. The content of "Compound 1 or salt thereof" is preferably about 1 - about 300 mg/mL, more preferably about 5 - about 150 mg/mL, furthermore preferably about 10-120 mg/mL, still furthermore preferably about 12 - about 100 mg/mL, particularly preferably about 20 - about 80 mg/mL, and most preferably about 25 - about 50 mg/mL in terms of Compound 1 hydrochloride. The term "in terms of Compound 1 hydrochloride" as used herein means calculating the amount of Compound 1, a pharmaceutically acceptable acid addition salt of Compound 1, hydrate or solvate thereof, or a mixture thereof as the amount of Compound 1 hydrochloride equimolar thereto.

The aqueous suspension preparation of the present invention may comprise a dispersant as appropriate.

The dispersant is not particularly limited as long as it is used as a pharmaceutical additive for a conventional oral solution or injection. Examples thereof include cellulose derivatives such as cellulose, hypromellose, hydroxypropylcellulose, methylcellulose, ethylcellulose, and carmellose sodium; alkyl alcohols such as polyvinyl alcohol; sucrose fatty acid esters such as sucrose myristate, sucrose laurate, and sucrose stearate;polyglyceryl fatty acid esters such as polyglyceryl myristate, polyglyceryl laurate, and polyglyceryl stearate; pyrrolidone derivatives such as polyvinylpyrrolidone; non-ionic surfactants such as Poloxamer 188, HCO-60, Polysorbate 80, and Polyoxyl 40 Stearate; ionic surfactants such as sodium lauryl sulfate. The dispersant is preferably cellulose derivatives, sucrose fatty acid esters and alkyl alcohols, more preferably cellulose derivatives and sucrose fatty acid esters, and furthermore preferably cellulose derivatives. Specific examples thereof preferably include methylcellulose, hypromellose, sucrose laurate, and hydroxypropylcellulose, more preferably methylcellulose, hypromellose, and hydroxypropylcellulose, and most preferably hypromellose. The dispersant may also be used alone or in mixture of two or more dispersants.

The content of the dispersant is preferably about 0.01 - about 40 mg/mL in the preparation, but is not limited thereto. More specifically, when the dispersant is cellulose derivatives such as methylcellulose, hypromellose, hydroxypropylcellulose or sucrose fatty acid esters such as sucrose laurate, the content thereof is preferably 0.1 - about 30

mg/mL, more preferably about 0.1 - about 20 mg/mL, furthermore preferably about 0.2 - about 20 mg/mL, and still furthermore preferably about 0.2 - about 10 mg/mL in the preparation, but is not limited thereto. Also, when the dispersant is alkyl alcohols such as polyvinyl alcohol, the content thereof is preferably about 0.1 - about 10 mg/mL and more preferably about 0.5 - about 5 mg/mL in the preparation, but is not limited thereto. When the dispersant is polyglycerin fatty acid esters, pyrrolidone derivatives, non-ionic surfactants or ionic surfactants, the content thereof is preferably about 0.01 - about 0.5 mg/mL, and more preferably about 0.01 - about 0.1 mg/mL in the preparation, but is not limited thereto.

The aqueous suspension preparation of the present invention may comprise a thickening agent as appropriate.

The thickening agent is not particularly limited as long as it is used as a pharmaceutical additive for a conventional oral solution or injection. Examples thereof include polysaccharides such as xanthan gum, guar gum, tamarind gum, gellan gum, carrageenan, sodium alginate, pectin, and agar; proteins such as casein and gelatin; acrylic acid polymers such as carboxy vinyl polymer. The thickening agent is preferably polysaccharides and acrylic acid polymers, more preferably polysaccharides. Specific example thereof preferably include xanthan gum, carrageenan, sodium alginate, pectin, and guar gum, more preferably xanthan gum, guar gum, carrageenan, and sodium alginate, furthermore preferably xanthan gum, guar gum, and sodium alginate, and still furthermore preferably xanthan gum and sodium alginate. In addition, the thickening agent may be used alone or in a mixture of two or more thickening agents.

The content of the thickening agent is preferably about 0.5 - about 20 mg/mL, more preferably about 1 - about 15 mg/mL, furthermore preferably about 2 - about 10 mg/mL in the preparation, but is not limited thereto.

The aqueous suspension preparation of the present invention may comprise a buffering agent as appropriate.

The buffering agent is not particularly limited as long as it is used as a pharmaceutical additive for a conventional oral solution or injection. Examples thereof include sodium salt, potassium salt, hydrochloride, and hydrate thereof. Examples of the sodium salt include sodium chloride, disodium phosphate, monosodium phosphate, sodium hydrogen carbonate, sodium carbonate, sodium citrate, sodium tartrate, sodium malate, sodium acetate, sodium lactate, and hydrate thereof. Examples of the potassium salt include potassium chloride, dipotassium phosphate, monopotassium phosphate, potassium hydrogen carbonate, potassium carbonate, potassium citrate, and hydrate thereof. Examples of the hydrochloride include glucosamine hydrochloride, guanidine hydrochloride, arginine hydrochloride, cysteine hydrochloride, and hydrate thereof. The buffering agent is preferably sodium salt, potassium salt, and hydrate thereof, and more preferably potassium salt and hydrate thereof. Specific examples thereof preferably include dipotassium phosphate, monopotassium phosphate, disodium phosphate, monosodium phosphate, potassium chloride, sodium chloride, sodium citrate, and hydrate thereof, more preferably dipotassium phosphate, monopotassium phosphate, disodium phosphate, monosodium phosphate, sodium chloride, and hydrate thereof, furthermore preferably dipotassium phosphate, monosodium phosphate, sodium chloride, and hydrate thereof. In addition, the buffering agent may be used alone or in a mixture of two or more buffering agents.

Dipotassium phosphate is the same compound as dipotassium hydrogen phosphate, and monopotassium phosphate is the same compound as potassium dihydrogen phosphate.

The content of the buffering agent is preferably about 0.01 - about 15 mg, more preferably about 0.05 - about 5 mg, furthermore preferably about 0.125 - about 0.85 mg, and particularly preferably about 0.2 - about 0.5 mg relative to 1 mg of Compound 1 or salt thereof in the preparation (the weight in terms of Compound 1 hydrochloride), but is not limited thereto. When the content of the buffering agent is less than 0.01 mg relative to 1 mg of Compound 1 or salt thereof in the preparation (the weight in terms of Compound 1 hydrochloride), there is the possibility to produce the reduced stability and administration feeling of Compound 1 or salt thereof because of the increased dissolution amount of Compound 1 or salt thereof to a solvent. When the content of the buffering agent exceeds 15 mg relative to 1 mg of Compound 1 or salt thereof in the preparation (the weight in terms of Compound 1 hydrochloride), there is the possibility to make the maintenance of the suspended state difficult because of the deposition of other additive such as a dispersant by salting out.

The aqueous suspension preparation of the present invention preferably uses a mixture of a polar solvent in an amount not affecting the administration feeling of the preparation and water or water itself as a solvent. The solvent therein is more preferably water.

Examples of the polar solvent which may be comprised in the aqueous suspension preparation of the present invention include an alcohol and preferably a polyalcohol, but are not limited thereto. Specific examples thereof preferably include propylene glycol, ethanol, glycerin, and polyethylene glycol, and more preferably propylene glycol. In addition, the polar solvent may be used alone or in a mixture of two or more polar solvents.

The content of the polar solvent in the aqueous suspension preparation of the present invention is not particularly limited as long as it does not affect the administration feeling of the preparation. The content of the polar solvent therein is preferably about 50% (w/v) or less, more preferably about 30% (w/v) or less, and furthermore preferably about 10% (w/v) or less.

The content of water in the aqueous suspension preparation of the present invention is not particularly limited as long as it is an amount for dissolving an additive other than Compound 1 or salt thereof to make the whole preparation clear. The content of water therein is preferably about 50% (w/v) or more, more preferably about 60% (w/v) or more,

and furthermore preferably about 70% (w/v) or more.

The aqueous suspension preparation of the present invention may comprise a preservative as appropriate.

The preservative is not particularly limited as long as it is used as a pharmaceutical additive for a conventional oral solution or injection. Examples thereof include a benzoic acid derivative such as methyl parahydroxybenzoate, ethyl parahydroxybenzoate, propyl parahydroxybenzoate, butyl parahydroxybenzoate, parahydroxybenzoic acid, benzoic acid, and sodium benzoate; a compound of a backbone comprising 3 or more carbon atoms with one to four carboxyl groups such as sorbic acid, potassium sorbate, sodium edetate and citric acid; and an alcohol such as glycerin, ethanol, 2-propanol and propylene glycol. The preservative is preferably a benzoic acid derivative. Specific examples thereof include methyl parahydroxybenzoate, ethyl parahydroxybenzoate, propyl parahydroxybenzoate, butyl parahydroxybenzoate and sodium benzoate, more preferably methyl parahydroxybenzoate, propyl parahydroxybenzoate and sodium benzoate, furthermore preferably methyl parahydroxybenzoate and sodium benzoate, and most preferably methyl parahydroxybenzoate. In addition, the preservative may be used alone or in a mixture of two or more preservatives.

The content of the preservative is preferably about 0.1 - about 10 mg/mL, more preferably about 0.2 - about 5 mg/mL, furthermore preferably about 0.25 - about 3 mg/mL, and particularly preferably about 0.5 - about 2.5 mg/mL in the preparation, but is not limited thereto.

The aqueous suspension preparation of the present invention may comprise a stabilizing agent as appropriate.

The stabilizing agent is not particularly limited as long as it is used as a pharmaceutical additive for a conventional oral solution or injection. Examples thereof include alcohol and oil. Examples of the alcohol include monohydric alcohols such as ethanol; polyhydric alcohols such as glycerin, propylene glycol, and polyethylene glycol; sugar alcohols such as sorbitol, erythritol, and mannitol. The stabilizing agent is preferably polyhydric alcohol. Specific examples thereof include preferably glycerin, polyethylene glycol, ethanol, sorbitol, erythritol, and propylene glycol, more preferably glycerin, polyethylene glycol, ethanol, and propylene glycol, and furthermore preferably propylene glycol. In addition, the stabilizing agent can be used alone or in mixture of two or more stabilizing agents.

The content of the stabilizing agent is preferably about 1 - about 500 mg/mL, more preferably about 10 - about 400 mg/mL, furthermore preferably about 30 - about 350 mg/mL, still furthermore preferably about 50 - about 300 mg/mL, and particularly preferably about 50 - about 150 mg/mL in the preparation in view of the effects on the administration feeling and stimulatability of the preparation, but is not limited thereto.

The dissolution amount of Compound 1 or salt thereof in the aqueous suspension preparation of the present invention can be measured. The dissolution amount of Compound 1 or salt thereof refers to the concentration of Compound 1 or salt thereof dissolved in the aqueous suspension preparation. The dissolution amount of Compound 1 or salt thereof is about 8 mg/mL or less. The dissolution amount of Compound 1 or salt thereof is preferably about 6 mg/mL or less, more preferably about 4 mg/mL or less, and furthermore preferably about 1 mg/mL.

The pH (at 25°C) of the aqueous suspension preparation of the present invention is preferably about 1.0 - about 10.0, more preferably about 2.0 - about 9.0, and furthermore preferably about 3.0 - about 8.0.

The aqueous suspension preparation of the present invention may comprise a pharmaceutical additive for a conventional oral solution and injection besides the above dispersant, thickening agent, buffering agent, preservative and stabilizing agent unless the effect of the present invention is lost. Examples of such additive include sweetening agent, coloring agent, and flavoring agent, but are not limited thereto.

The sweetening agent is not particularly limited as long as it is used as a pharmaceutical additive for a conventional oral solution or injection. Examples thereof include sucralose, sucrose, liquid sugar, fructose, sorbitol, xylitol, erythritol, trehalose, maltitol, and acesulfame potassium. The sweetening agent is preferably sucralose, sorbitol, and erythritol, more preferably sucralose and erythritol, and furthermore preferably sucralose. In addition, the sweetening agent may be used alone or in mixture of two or more sweetening agents.

The coloring agent is not particularly limited as long as it is used as a pharmaceutical additive for a conventional oral solution or injection. Examples thereof include tar dye, Yellow No.5, and caramel. In addition, the coloring agent may be used alone or in a mixture of two or more coloring agents.

The flavoring agent is not particularly limited as long as it is used as a pharmaceutical additive for a conventional oral solution or injection. Examples thereof include medical essence, lemon oil, orange oil, and peach oil. In addition, the flavoring agent may be used alone or in a mixture of two or more flavoring agents.

When the aqueous suspension preparation of the present invention comprises a thickening agent, the circularity of the suspended particle is preferably about 0.960 or less. The circularity of the suspended particle in the preparation comprising a thickening agent is more preferably about 0.700 - about 0.960, and furthermore preferably about 0.800 - about 0.960.

When the aqueous suspension preparation of the present invention comprises no thickening agent, the circularity of the suspended particle is preferably about 0.945 or less. The circularity of the suspended particle in the preparation comprising no thickening agent is more preferably about 0.700 - about 0.945, and furthermore preferably about 0.800 - about 0.945.

The term "when the preparation comprises no thickening agent" as used herein means "when the preparation

is substantially free of thickening agent". The term "when the preparation is substantially free of thickening agent" comprises "the case that the preparation comprises a trace amount of thickening agent which does not produce the effect of thickening agent" besides the case that the content of the thickening agent in the preparation is literally zero. Examples of the effect of thickening agent include a thickening effect (an effect for increasing the viscosity of the preparation), an effect for enhancing stability of the preparation, and a protective colloidal effect (an effect for stably dispersing the fine particle in the preparation). The situation that none of these effects is produced as compared to the preparation in which the content of the thickening agent is zero is referred to as "the preparation is substantially free of thickening agent". The content of the thickening agent in the preparation which "is substantially free of thickening agent" can be varied depending on each thickening agent and the feature of the added preparation itself. Examples thereof include less than about 0.1 mg/mL, but are not limited thereto. The content of the thickening agent in the preparation which "is substantially free of thickening agent" is preferably less than about 0.05 mg/mL, and more preferably less than about 0.01 mg/mL.

The term "when the preparation comprises a thickening agent" as used herein has an meaning other than the above term "when the preparation substantively comprises no thickening agent".

The aqueous suspension preparation of the present invention may comprise a suspended particle other than the suspended particle of "Compound 1, a pharmaceutically acceptable acid addition salt thereof, or a mixture thereof" (Compound 1 or salt thereof) as "suspended particle". Such aqueous suspension preparation is preferably a preparation wherein the amount of the suspended particle other than that of Compound 1 or salt thereof is an amount which does not affect the measurement of any factors such as the circularity in such preparation (including a preparation in which the amount is zero), and more preferably a preparation obtained by excluding suspended particle other than that of Compound 1 or salt thereof.

The median diameter of the suspended particle of Compound 1 or salt thereof in the aqueous suspension preparation of the present invention is preferably about 0.1 - about 30 pm, more preferably about 0.5 - about 20 μm, and furthermore preferably about 1 - about 10 pm, but is not limited thereto.

The suspended particle of Compound 1 or salt thereof whose median diameter is about 0.1 - about 30 μm can be produced by dry milling Compound 1 or salt thereof using a mill such as jet mill and hammer mill, but the present invention is not limited thereto. Also, the suspended particle can be produced by adding Compound 1 or salt thereof into a solvent, and then wet-milling the resulting product using a machine such as homomixer, high-speed rotation type disperser, high-pressure homogenizer and bead mill. In addition, the suspended particle can be produced by a commonly-used recrystallization technique of Compound 1 or salt thereof besides the method for milling Compound 1 or salt thereof.

As Compound 1 or salt thereof for using at the time of the manufacture of the aqueous suspension preparation of the present invention, Compound 1 or salt thereof with a median diameter of about 0.1 - about 30 μm produced by an operation such as dry-milling, wet-milling and recrystallization technique can be used, but is not limited thereto. Even if Compound 1 or salt thereof does not have a median diameter of about 0.1 - about 30 μm at the time of the manufacture of the aqueous suspension preparation of the present invention, the suspended particle of Compound 1 or salt thereof with about 0.1 - about 30 μm can be produced by an operation such as wet-milling in the manufacture step.

The aqueous suspension preparation of the present invention can be prepared by a preparation process of a conventional oral solution or injection medicine. For example, the aqueous suspension preparation of the present invention can be prepared by the process comprising the following steps (1)-(3), but the present invention is not limited thereto.

(1) An additive other than Compound 1 or salt thereof is added to a solvent and dissolved.
(2) To the solution prepared in the above (1) is added dry-milled Compound 1 or salt thereof and mixed.
(3) As appropriate, the clump of Compound 1 or salt thereof added in the above (2) is loosened with a machine such as homomixer, stirring machine and high-speed rotation type disperser (wet-milling) or the clump is stirred or dispersed, and thus the aqueous suspension preparation of the present invention can be prepared.

The aqueous suspension preparation of the present invention can be also prepared by the process comprising the following steps (1)-(5), but the present invention is not limited thereto.

(1) An additive other than Compound 1 or salt thereof and a thickening agent is added to a solvent and dissolved.
(2) To the solution prepared in the above (1) is added the dry-milled Compound 1 or salt thereof and mixed to prepare Solution A.
(3) As appropriate, the clump of Compound 1 or salt thereof added in the above (2) is loosened with a machine such as homomixer, stirring machine and high-speed rotation type disperser (wet-milling) or the clump is stirred or dispersed to homogeneously suspend Compound 1 or salt thereof.
(4) Separately, in another container, an additive other than Compound 1 or salt thereof and a dispersant is added to a solvent (when the preparation comprises a thickening agent, the thickening agent is also added) and dissolved

to prepare Solution B.

(5) The aqueous suspension preparation of the present invention can be prepared by mixing Solution A and Solution B prepared in the above in an appropriate amount.

When an additive is added to a solvent and dissolved in the manufacture of the aqueous suspension preparation of the present invention, the additive can also be dissolved using a heated solvent. Also, the additive is preliminarily dissolved in a solvent such as propylene glycol, ethanol and glycerin and the resulting solution may be mixed to a solvent. These processes are helpful when an additive with low solubility in a solvent is dissolved to the solvent. Examples of the additive with low solubility in a solvent include a preservative, but are not limited thereto.

The aqueous suspension preparation of the present invention have no specific disadvantage even if the preparation has air bubbles. The preparation process may be modified so that the preparation has no air bubble. Specifically, the aqueous suspension preparation of the present invention in which no air bubble is visually contained can be prepared, for example, by appropriately modifying any preparation condition such as processing time, processing strength, liquid temperature of processed product and indoor pressure in the wet-milling, stirring or dispersion step, but the present invention is not limited thereto. Even if air bubbles are contained in the preparation in the preparation process, the contained air bubbles can be removed from the aqueous suspension preparation of the present invention by the introduction of a step such as reduced pressure step and stirring step.

The storage container of the aqueous suspension preparation of the present invention is not particularly limited as long as it is a storage container for a conventional oral solution or injection medicine. Examples thereof include a vial, an ample, a glass bottle, a polyethylene bottle and a container divided by aluminum multilayer film.

The aqueous suspension preparation of the present invention can be prepared in an appropriate concentration depending on a factor such as dosage, and administered by adjusting the dosage as appropriate.

Even if the aqueous suspension preparation of the present invention is administered orally, the preparation has little effect on gastrointestinal tract motility and environment in the gastrointestinal tract. As a result, the aqueous suspension preparation of the present invention can also be administered in emergency situations.

The aqueous suspension preparation of the present invention is preferably that Compound 1 or salt thereof in suspended state limits the movements of many water molecules in the preparation. In such state, the degree of the limitation can be evaluated by the measurement of the relaxation time of protons in water molecules, for example, with a pulse NMR particle boundary evaluation apparatus. Specifically, the aqueous suspension preparation comprising Compound 1 or salt thereof in a content of 40 mg/mL (in terms of Compound 1 hydrochloride) was measured using pulse NMR particle boundary evaluation apparatus and the calculated Rsp is preferably about 0.25 or more. The Rsp is more preferably about 0.25 - about 2.0, and furthermore preferably about 0.25 - about 1.5.

The aqueous suspension preparation of the present invention preferably has flowability which does not affect usability, administration feeling and manufacturability, but the present invention is not limited thereto. For example, the flowability which does not affect usability, administration feeling and manufacturability preferably means that a preparation starts to flow within 1 minute after a test tube filled with the preparation is reversed, more preferably that a preparation starts to flow within 30 seconds after a test tube filled with the preparation is reversed, and furthermore preferably that a preparation starts to flow within 10 seconds after a test tube filled with the preparation is reversed, but the present invention is not limited thereto.

## EXAMPLES

Hereinafter, the present invention is explained in more detail in Examples, Comparative Examples, Test Examples, but should not be limited thereto.

In the following Examples and Comparative Examples, Compound 1 hydrochloride prepared according to the process of Patent Document 2 was milled to a particle with a median diameter of about 1.0 - about 4.0 μm by dry-milling and the resulting particle was used.

In the following Examples and Comparative Examples, the following hypromellose, hydroxypropylcellulose and sucrose laurate were used, but the present invention are not limited thereto.

- hypromellose: TC-5R (manufactured by Shin-Etsu Chemical Co., Ltd.)
- hydroxypropylcellulose: NISSO HPC-L (manufactured by Nippon Soda Co., Ltd.)
- sucrose laurate: RYOTO Sugar Ester L-1695 (manufactured by Mitsubishi-Chemical Foods Corporation, RYOTO is registered trademark)

### Examples 1-5

According to the following procedure, the preparations of Examples 1-5 were prepared from Compound 1

hydrochloride, an additive and a solvent in the amounts shown in Table below.

Dipotassium phosphate was added to purified water and dissolved. The solution was then warmed to 70°C and methyl parahydroxybenzoate was added thereto and dissolved. The solution was cooled to room temperature, and then hypromellose, hydroxypropylcellulose, sucrose laurate, polyvinyl alcohol or methylcellulose was added thereto and dissolved. To the solution was then added Compound 1 hydrochloride, and the mixture was dispersed with Precision Dispersion/Emulsification machine: CLEARMIX CLM-0.8S (M Technique Co., Ltd.) at 8000 rpm for 10 minutes to prepare the preparations of Examples 1-5.

| Formulation amount (Content) | Example | | |
|---|---|---|---|
| | 1 | 2 | 3 |
| Compound 1 hydrochloride | 8.0 g (40 mg/mL) | 8.0 g (40 mg/mL) | 8.0 g (40 mg/mL) |
| hypromellose | 0.2 g (1 mg/mL) | - | - |
| hydroxypropylcellul ose | - | 0.2 g (1 mg/mL) | - |
| sucrose laurate | - | - | 0.2 g (1 mg/mL) |
| dipotassium phosphate | 3.12 g (90 mM) | 3.12 g (90 mM) | 3.12 g (90 mM) |
| methyl parahydroxybenzoate | 0.1 g (0.5 mg/mL) | 0.1 g (0.5 mg/mL) | 0.1g (0.5 mg/mL) |
| purified water | 200 mL in total | 200 mL in total | 200 mL in total |
| Formulation amount (Content) | Example | | |
| | 4 | 5 | |
| Compound 1 hydrochloride | 8.0 g (40 mg/mL) | 8.0 g (40 mg/mL) | |
| polyvinyl alcohol | 0.2 g (1 mg/mL) | - | |
| methylcellulose | - | 0.2 g (1 mg/mL) | |
| dipotassium | 3.12 g | 3.12 g | |
| phosphate | (90 mM) | (90 mM) | |
| methyl parahydroxybenzoate | 0.1 g (0.5 mg/mL) | 0.1 g (0.5 mg/mL) | |
| purified water | 200 mL in total | 200 mL in total | |

Example 6

According to the following procedure, the preparation of Example 6 was prepared from Compound 1 hydrochloride, an additive and a solvent in the amounts shown in Table below.

Dipotassium phosphate was added to purified water and dissolved. Propylene glycol and methyl parahydroxybenzoate were then weighed, methyl parahydroxybenzoate was dissolved in propylene glycol in another container, and then the solution was added to purified water. Hypromellose was then added thereto and dissolved. To the solution was then added Compound 1 hydrochloride, and the mixture was dispersed with Precision Dispersion/Emulsification machine: CLEARMIX CLM-0.8S (M Technique Co., Ltd.) at 8000 rpm for 10 minutes to prepare the preparation of Example 6.

| Formulation amount (Content) | Example |
|---|---|
| | 6 |
| Compound 1 hydrochloride | 8.0 g (40 mg/mL) |
| hypromellose | 0.2 g (1 mg/mL) |
| propylene glycol | 10.0 g (50 mg/mL) |
| dipotassium phosphate | 3.12 g (90 mM) |
| methyl parahydroxybenzoate | 0.5 g (2.5 mg/mL) |
| purified water | 200 mL in total |

Examples 7-10

According to the following procedure, the preparations of Examples 7-10 were prepared from Compound 1 hydrochloride, an additive and a solvent in the amounts shown in Table below.

Dipotassium phosphate was added to purified water and dissolved. The solution was then warmed to 70°C and methyl parahydroxybenzoate was added thereto and dissolved. The solution was cooled to room temperature, and then hypromellose or Poloxamer 188 was added thereto and dissolved. To the solution was then added Compound 1 hydrochloride, and the mixture was dispersed with Precision Dispersion/Emulsification machine: CLEARMIX CLM-0.8S (M Technique Co., Ltd.) at 8000 rpm for 10 minutes to prepare the preparations of Examples 7-10.

| Formulation amount (Content) | Example | | |
|---|---|---|---|
| | 7 | 8 | 9 |
| Compound 1 hydrochloride | 8.0 g (40 mg/mL) | 8.0 g (40 mg/mL) | 8.0 g (40 mg/mL) |
| hypromellose | 0.04 g (0.2 mg/mL) | 0.08 g (0.4 mg/mL) | 0.12 g (0.6 mg/mL) |
| Poloxamer 188 | 0.16 g (0.8 mg/mL) | 0.12 g (0.6 mg/mL) | 0.08 g (0.4 mg/mL) |
| dipotassium phosphate | 3.12 g (90 mM) | 3.12 g (90 mM) | 3.12 g (90 mM) |
| methyl parahydroxybenzoate | 0.1 g (0.5 mg/mL) | 0.1 g (0.5 mg/mL) | 0.1 g (0.5 mg/mL) |
| purified water | 200 mL in total | 200 mL in total | 200 mL in total |
| Formulation amount (Content) | Example | | |
| | 10 | | |
| Compound 1 | 8.0 g | | |
| hydrochloride | (40 mg/mL) | | |
| hypromellose | 0.16 g (0.8 mg/mL ) | | |
| Poloxamer 188 | 0.04 g (0.2 mg/mL) | | |
| dipotassium phosphate | 3.12 g (90 mM) | | |
| methyl parahydroxybenzoate | 0.1 g (0.5 mg/mL) | | |
| purified water | 200 mL in total | | |

Examples 11 and 12

According to the following procedure, the preparations of Examples 11 and 12 were prepared from Compound 1 hydrochloride, an additive and a solvent in the amounts shown in Table below.

Dipotassium phosphate was added to purified water and dissolved. The solution was then warmed to 70°C and methyl parahydroxybenzoate was added thereto and dissolved. The solution was cooled to room temperature, and then hypromellose was added thereto and dissolved. To the solution was then added Compound 1 hydrochloride, and the mixture was dispersed with Precision Dispersion/Emulsification machine: CLEARMIX CLM-0.8S (M Technique Co., Ltd.) at 8000 rpm for 10 minutes to prepare the preparations of Examples 11 and 12.

| Formulation amount (Content) | Example | |
|---|---|---|
| | 11 | 12 |
| Compound 1 hydrochloride | 24 g (120 mg/mL) | 16 g (80 mg/mL) |
| hypromellose | 0.2 g (1 mg/mL) | 0.2 g (1 mg/mL) |
| dipotassium phosphate | 3.12 g (90 mM) | 3.12 g (90 mM) |
| methyl parahydroxybenzoate | 0.1 g (0.5 mg/mL) | 0.1 g (0.5 mg/mL) |
| purified water | 200 mL in total | 200 mL in total |

Example 13

According to the following procedure, the preparation of Example 13 was prepared from Compound 1 hydrochloride, an additive and a solvent in the amounts shown in Table below.

(1) Preparation of Solution A

Hypromellose was added to purified water and dissolved. Dipotassium phosphate was then added thereto and dissolved. Propylene glycol and methyl parahydroxybenzoate were then weighed, methyl parahydroxybenzoate was dissolved in propylene glycol in another container, and then the solution were added to purified water containing dipotassium phosphate. To the solution was then added Compound 1 hydrochloride, and the mixture was dispersed with Precision Dispersion/Emulsification machine: CLEARMIX CLM-0.8S (M Technique Co., Ltd.) at 8000 rpm for 10 minutes to prepare Solution A.

(2) Preparation of Solution B

Dipotassium phosphate was added to purified water and dissolved. Propylene glycol and methyl parahydroxybenzoate were then weighed, methyl parahydroxybenzoate was dissolved in propylene glycol in another container, and then the solution were added to purified water containing dipotassium phosphate. The solution was then warmed to 70°C, and xanthan gum was added thereto and dissolved. The solution was cooled to room temperature to prepare Solution B.

(3) Preparation of Example 13

Solution A and Solution B were mixed in a mass ratio of 1:1 to prepare the preparation of Example 13.

| Formulation amount (Content) | Example 13 | |
|---|---|---|
| | Solution A | Solution B |
| Compound 1 hydrochloride | 8.0 g (80 mg/mL) | - |
| hypromellose | 2.0 g (20 mg/mL) | - |
| propylene glycol | 5.0 g (50 mg/mL) | 5.0 g (50 mg/mL) |
| dipotassium phosphate | 1.56 g (90 mM) | 1.56 g (90 mM) |
| methyl parahydroxybenzoate | 0.25 g (2.5 mg/mL) | 0.25 g (2.5 mg/mL) |
| xanthan gum | - | 0.8 g (8 mg/mL) |
| purified water | 100 mL in total | 100 mL in total |

The formulation amount and content of each ingredient in the preparations of the above Examples are shown in Table below.

| Formulation amount (Content) | Example |
|---|---|
| | 13 |
| Compound 1 hydrochloride | 8.0 g (40 mg/mL) |
| hypromellose | 2.0 g (10 mg/mL) |
| propylene glycol | 10.0 g (50 mg/mL) |
| dipotassium phosphate | 3.12 g (90 mM) |
| methyl parahydroxybenzoate | 0.5 g (2.5 mg/mL) |
| xanthan gum | 0.8 g (4 mg/mL) |
| purified water | 200 mL in total |

Examples 14 and 15

According to the following procedure, the preparations of Examples 14 and 15 were prepared from Compound 1 hydrochloride, an additive and a solvent in the amounts shown in Table below.

(1) Preparation of Solution A

Hypromellose was added to purified water and dissolved. Monopotassium phosphate and dipotassium phosphate were then added thereto and dissolved. The solution was then warmed to 70°C and methyl parahydroxybenzoate was added thereto and dissolved. The solution was then cooled to room temperature, and then to the solution was added Compound 1 hydrochloride, and the mixture was dispersed with Precision Dispersion/Emulsification machine: CLEARMIX CLM-0.8S (M Technique Co., Ltd.) at 8000 rpm for 10 minutes to prepare Solution A.

(2) Preparation of Solution B

Monopotassium phosphate and dipotassium phosphate were added to purified water and dissolved. The solution was then warmed to 70°C and methyl parahydroxybenzoate was added thereto and dissolved. Xanthan gum was then added thereto and dissolved. The solution was then cooled to room temperature to prepare Solution B.

(3) Preparation of Examples 14 and 15

Solution A and Solution B were mixed in a mass ratio of 1:1 to prepare the preparations of Examples 14 and 15.

| Formulation amount (Content) | Example 14 | |
|---|---|---|
| | Solution A | Solution B |
| Compound 1 hydrochloride | 4.0 g (40 mg/mL) | - |
| hypromellose | 0.4 g (4.0 mg/mL) | - |
| monopotassium phosphate | 0.41 g (30 mM) | 0.41 g (30 mM) |
| dipotassium phosphate | 1.22 g (70 mM) | 1.22 g (70 mM) |
| methyl parahydroxybenzoate | 0.2 g (2.0 mg/mL) | 0.2 g (2.0 mg/mL) |
| xanthan gum | - | 0.8 g (8.0 mg/mL) |
| purified water | 100 mL in total | 100 mL in total |
| Formulation amount (Content) | Example 15 | |
| | Solution A | Solution B |
| Compound 1 hydrochloride | 4.0 g (40 mg/mL) | - |
| hypromellose | 1.0 g (10.0 mg/mL) | - |
| monopotassium phosphate | 0.41 g (30 mM) | 0.41 g (30 mM) |
| dipotassium phosphate | 1.22 g (70 mM) | 1.22 g (70 mM) |
| methyl parahydroxybenzoate | 0.2 g (2.0 mg/mL) | 0.2 g (2.0 mg/mL) |
| xanthan gum | - | 0.8 g (8.0 mg/mL) |
| purified water | 100 mL in total | 100 mL in total |

The formulation amount and content of each ingredient in the preparations of the above Examples are shown in Table below.

| Formulation amount (Content) | Example | |
|---|---|---|
| | 14 | 15 |
| Compound 1 hydrochloride | 4.0 g (20 mg/mL) | 4.0 g (20 mg/mL) |

(continued)

| Formulation amount (Content) | Example | |
|---|---|---|
| | 14 | 15 |
| hypromellose | 0.4 g (2.0 mg/mL) | 1.0 g (5.0 mg/mL) |
| monopotassium phosphate | 0.82 g (30 mM) | 0.82 g (30 mM) |
| dipotassium phosphate | 2.44 g (70 mM) | 2.44 g (70 mM) |
| methyl parahydroxybenzoate | 0.4 g (2.0 mg/mL) | 0.4 g (2.0 mg/mL) |
| xanthan gum | 0.8 g (4.0 mg/mL) | 0.8 g (4.0 mg/mL) |
| purified water | 200 mL in total | 200 mL in total |

Examples 16-19

According to the following procedure, the preparations of Examples 16-19 were prepared from Compound 1 hydrochloride, an additive and a solvent in the amounts shown in Table below.

(1) Preparation of Solution A

Hypromellose was added to purified water and dissolved. Dipotassium phosphate was then added and dissolved. Propylene glycol and methyl parahydroxybenzoate were then weighed, methyl parahydroxybenzoate was dissolved in propylene glycol in another container, and then the solution was added to purified water containing dipotassium phosphate. To the solution was then added Compound 1 hydrochloride, and the mixture was dispersed with Precision Dispersion/Emulsification machine: CLEARMIX CLM-0.8S (M Technique Co., Ltd.) at 8000 rpm for 10 minutes to prepare Solution A.

(2) Preparation of Solution B

Dipotassium phosphate was dissolved in purified water. Propylene glycol and methyl parahydroxybenzoate were weighed, methyl parahydroxybenzoate was dissolved in propylene glycol in another container, and then the solution was added to purified water containing dipotassium phosphate. The solution was then warmed to 70°C, and xanthan gum and guar gum were added thereto and dissolved. The solution was then cooled to room temperature to prepare Solution B.

(3) Preparation of Examples 16-19

Solution A and Solution B were mixed in a mass ratio of 1:1 to prepare the preparations of Examples 16-19. s

| Formulation amount (Content) | Example 16 | |
|---|---|---|
| | Solution A | Solution B |
| Compound 1 hydrochloride | 8.0 g (80 mg/mL) | - |
| hypromellose | 2.0 g (20 mg/mL) | - |
| propylene glycol | 5.0 g (50 mg/mL) | 5.0 g (50 mg/mL) |
| dipotassium phosphate | 1.56 g (90 mM) | 1.56 g (90 mM) |
| methyl parahydroxybenzoate | 0.25 g (2.5 mg/mL) | 0.25 g (2.5 mg/mL) |
| xanthan gum | - | 0.8 g (8.0 mg/mL) |
| guar gum | - | 0.6 g (6.0 mg/mL) |
| purified water | 100 mL in total | 100 mL in total |

(continued)

| Formulation amount (Content) | Example 17 | |
|---|---|---|
| | Solution A | Solution B |
| Compound 1 hydrochloride | 8.0 g (80 mg/mL) | - |
| hypromellose | 2.0 g (20 mg/mL) | - |
| propylene glycol | 5.0 g (50 mg/mL) | 5.0 g (50 mg/mL) |
| dipotassium phosphate | 1.56 g (90 mM) | 1.56 g (90 mM) |
| methyl parahydroxybenzoate | 0.25 g (2.5 mg/mL) | 0.25 g (2.5 mg/mL) |
| xanthan gum | - | 0.8 g (8.0 mg/mL) |
| guar gum | - | 1.0 g (10.0 mg/mL) |
| purified water | 100 mL in total | 100 mL in total |
| Formulation amount (Content) | Example 18 | |
| | Solution A | Solution B |
| Compound 1 hydrochloride | 8.0 g (80 mg/mL) | - |
| hypromellose | 2.0 g (20 mg/mL) | - |
| propylene glycol | 5.0 g (50 mg/mL) | 5.0 g (50 mg/mL) |
| dipotassium phosphate | 1.56 g (90 mM) | 1.56 g (90 mM) |
| methyl parahydroxybenzoate | 0.25 g (2.5 mg/mL) | 0.25 g (2.5 mg/mL) |
| xanthan gum | - | 0.4 g (4.0 mg/mL) |
| guar gum | - | 0.6 g (6.0 mg/mL) |
| purified water | 100 mL in total | 100 mL in total |
| Formulation amount (Content) | Example 19 | |
| | Solution A | Solution B |
| Compound 1 hydrochloride | 8.0 g (80 mg/mL) | - |
| hypromellose | 2.0 g (20 mg/mL) | - |
| propylene glycol | 5.0 g (50 mg/mL) | 5.0 g (50 mg/mL) |
| dipotassium phosphate | 1.56 g (90 mM) | 1.56 g (90 mM) |
| methyl parahydroxybenzoate | 0.25 g (2.5 mg/mL) | 0.25 g (2.5 mg/mL) |
| xanthan gum | - | 0.4 g (4.0 mg/mL) |
| guar gum | - | 1.0 g (10.0 mg/mL) |
| purified water | 100 mL in total | 100 mL in total |

The formulation amount and content of each ingredient in the preparations of the above Examples are shown in Table below.

| Formulation amount (Content) | Example | | |
|---|---|---|---|
| | 16 | 17 | 18 |
| Compound 1 hydrochloride | 8.0 g (40 mg/mL) | 8.0 g (40 mg/mL) | 8.0 g (40 mg/mL) |
| hypromellose | 2.0 g (10 mg/mL) | 2.0 g (10 mg/mL) | 2.0 g (10 mg/mL) |
| propylene glycol | 10.0 g (50 mg/mL) | 10.0 g (50mg/mL) | 10.0 g (50mg/mL) |
| dipotassium phosphate | 3.12 g (90 mM) | 3.12 g (90 mM) | 3.12 g (90 mM) |
| methyl parahydroxybenzoate | 0.5 g (2.5 mg/mL) | 0.5 g (2.5 mg/mL) | 0.5 g (2.5 mg/mL) |
| xanthan gum | 0.8 g (4.0 mg/mL) | 0.8 g (4.0 mg/mL) | 0.4 g (2.0 mg/mL) |
| guar gum | 0.6 g (3.0 mg/mL) | 1.0 g (5.0 mg/mL) | 0.6 g (3.0 mg/mL) |
| purified water | 200 mL in total | 200 mL in total | 200 mL in total |

| Formulation amount (Content) | Example |
|---|---|
| | 19 |
| Compound 1 hydrochloride | 8.0 g (40 mg/mL) |
| hypromellose | 2.0 g (10 mg/mL) |
| propylene glycol | 10.0g (50mg/mL) |
| dipotassium phosphate | 3.12 g (90 mM) |
| methyl parahydroxybenzoate | 0.5 g (2.5 mg/mL) |
| xanthan gum | 0.4 g (2.0 mg/mL) |
| guar gum | 1.0 g (5.0 mg/mL) |
| purified water | 200 mL in total |

Examples 20-23

According to the following procedure, the preparations of Examples 20-23 were prepared from Compound 1 hydrochloride, an additive and a solvent in the amounts shown in Table below.

(1) Preparation of Solution A

Hypromellose was added to purified water and dissolved. Dipotassium phosphate was then added thereto and dissolved. Propylene glycol and methyl parahydroxybenzoate were then weighed, methyl parahydroxybenzoate was dissolved in propylene glycol in another container, and then the solution was added to purified water containing dipotassium phosphate. To the solution was then added Compound 1 hydrochloride, and the mixture was dispersed with

Precision Dispersion/Emulsification machine: CLEARMIX CLM-0.8S (M Technique Co., Ltd.) at 8000 rpm for 10 minutes to prepare Solution A.

(2) Preparation of Solution B

Dipotassium phosphate was added to purified water and dissolved. Propylene glycol and methyl parahydroxybenzoate were weighed, methyl parahydroxybenzoate was dissolved in propylene glycol in another container, and then the solution was added to purified water containing dipotassium phosphate. The solution was then warmed to 70°C, and xanthan gum was added thereto and dissolved. The solution was then cooled to room temperature to prepare Solution B.

(3) Preparation of Examples 20-23

Solution A and Solution B were mixed in a mass ratio of 1:1 to prepare the preparations of Examples 20-23.

| Formulation amount (Content) | Example 20 | |
|---|---|---|
| | Solution A | Solution B |
| Compound 1 hydrochloride | 8.0 g (80 mg/mL) | - |
| hypromellose | 0.2 g (2 mg/mL) | - |
| propylene glycol | 5.0 g (50 mg/mL) | 5.0 g (50 mg/mL) |
| dipotassium phosphate | 1.56 g (90 mM) | 1.56 g (90 mM) |
| methyl parahydroxybenzoate | 0.25 g (2.5 mg/mL) | 0.25 g (2.5 mg/mL) |
| xanthan gum | - | 0.8 g (8.0 mg/mL) |
| purified water | 100 mL in total | 100 mL in total |
| Formulation amount (Contained amount) | Example 21 | |
| | Solution A | Solution B |
| Compound 1 hydrochloride | 8.0 g (80 mg/mL) | |
| hypromellose | 1.0 g (10 mg/mL) | - |
| propylene glycol | 5.0 g (50 mg/mL) | 5.0 g (50 mg/mL) |
| dipotassium phosphate | 1.56 g (90 mM) | 1.56 g (90 mM) |
| methyl parahydroxybenzoate | 0.25 g (2.5 mg/mL) | 0.25 g (2.5 mg/mL) |
| xanthan gum | - | 0.8 g (8.0 mg/mL) |
| purified water | 100 mL in total | 100 mL in total |
| Formulation amount (Content) | Example 22 | |
| | Solution A | Solution B |
| Compound 1 hydrochloride | 8.0 g (80 mg/mL) | |
| hypromellose | 3.0 g (30 mg/mL) | - |
| propylene glycol | 5.0 g (50 mg/mL) | 5.0 g (50 mg/mL) |
| dipotassium phosphate | 1. 56 g (90 mM) | 1.56 g (90 mM) |
| methyl parahydroxybenzoate | 0.25 g (2.5 mg/mL) | 0.25 g (2.5 mg/mL) |
| xanthan gum | - | 0.8 g (8.0 mg/mL) |
| purified water | 100 mL in total | 100 mL in total |
| Formulation amount (Content) | Example 23 | |
| | Solution A | Solution B |
| Compound 1 hydrochloride | 8.0 g (80 mg/mL) | - |

(continued)

| Formulation amount (Content) | Example 23 | |
|---|---|---|
| | Solution A | Solution B |
| hypromellose | 4.0 g (40 mg/mL) | - |
| propylene glycol | 5.0 g (50 mg/mL) | 5.0 g (50 mg/mL) |
| dipotassium phosphate | 1.56 g (90 mM) | 1.56 g (90 mM) |
| methyl parahydroxybenzoate | 0.25 g (2.5 mg/mL) | 0.25 g (2.5 mg/mL) |
| xanthan gum | - | 0.8 g (8.0 mg/mL) |
| purified water | 100 mL in total | 100 mL in total |

The formulation amount and content of each ingredient in the preparations of the above Examples are shown in Table below.

| Formulation amount (Content) | Example | | |
|---|---|---|---|
| | 20 | 21 | 22 |
| Compound 1 hydrochloride | 8.0 g (40 mg/mL) | 8.0 g (40 mg/mL) | 8.0 g (40 mg/mL) |
| hypromellose | 0.2 g (1 mg/mL) | 1.0 g (5 mg/mL) | 3.0 g (15 mg/mL) |
| propylene glycol | 10.0 g (50 mg/mL) | 10.0 g (50 mg/mL) | 10.0 g (50 mg/mL) |
| dipotassium phosphate | 3.12 g (90 mM) | 3.12 g (90 mM) | 3.12 g (90 mM) |
| methyl parahydroxybenzoate | 0.5 g (2.5 mg/mL) | 0.5 g (2.5 mg/mL) | 0.5 g (2.5 mg/mL) |
| xanthan gum | 0.8 g (4.0 mg/mL) | 0.8 g (4.0 mg/mL) | 0.8 g (4.0 mg/mL) |
| purified water | 200 mL in total | 200 mL in total | 200 mL in total |
| Formulation amount (Content) | Example | | |
| | 23 | | |
| Compound 1 hydrochloride | 8.0 g (40 mg/mL) | | |
| hypromellose | 4.0 g (20 mg/mL) | | |
| propylene glycol | 10.0 g (50 mg/mL) | | |
| dipotassium phosphate | 3.12 g (90 mM) | | |
| methyl parahydroxybenzoate | 0.5 g (2.5 mg/mL) | | |
| xanthan gum | 0.8 g (4.0 mg/mL) | | |
| purified water | 200 mL in total | | |

Examples 24-27

According to the following procedure, the preparations of Examples 24-27 were prepared from Compound 1 hydrochloride, an additive and a solvent in the amounts shown in Table below.

(1) Preparation of Solution A

Hypromellose was added to purified water and dissolved. Dipotassium phosphate was then added thereto and dissolved. Propylene glycol and methyl parahydroxybenzoate were weighed, methyl parahydroxybenzoate was dissolved in propylene glycol in another container, and then the solution was added to purified water containing dipotassium phosphate. To the solution was then added Compound 1 hydrochloride, and the mixture was dispersed with Precision Dispersion/Emulsification machine: CLEARMIX CLM-0.8S (M Technique Co., Ltd.) at 8000 rpm for 10 minutes to prepare Solution A.

(2) Preparation of Solution B

Dipotassium phosphate was added to purified water and dissolved. Propylene glycol and methyl parahydroxybenzoate were weighed, methyl parahydroxybenzoate was dissolved in propylene glycol in another container, and then the solution was added to purified water containing dipotassium phosphate. The solution was then warmed to 70°C, and one or two ingredients selected from the group consisting of xanthan gum, sodium alginate and carboxy vinyl polymer were added thereto and dissolved. The solution was then cooled to room temperature to prepare Solution B.

(3) Preparation of Examples 24-27

Solution A and Solution B were mixed in a mass ratio of 1:1 to prepare the preparations of Examples 24-27.

| Formulation amount (Content) | Example 24 | |
|---|---|---|
| | Solution A | Solution B |
| Compound 1 hydrochloride | 8.0 g (80 mg/mL) | |
| hypromellose | 2.0 g (20 mg/mL) | - |
| propylene glycol | 5.0 g (50 mg/mL) | 5.0 g (50 mg/mL) |
| dipotassium phosphate | 1.56 g (90 mM) | 1.56 g (90 mM) |
| methyl parahydroxybenzoate | 0.25 g (2.5 mg/mL) | 0.25 g (2.5 mg/mL) |
| xanthan gum | - | 0.8 g (8.0 mg/mL) |
| sodium alginate | - | 0.4 g (4.0 mg/mL) |
| purified water | 100 mL in total | 100 mL in total |
| Formulation amount (Content) | Example 25 | |
| | Solution A | Solution B |
| Compound 1 hydrochloride | 8.0 g (80 mg/mL) | |
| hypromellose | 2.0 g (20 mg/mL) | - |
| propylene glycol | 5.0 g (50 mg/mL) | 5.0 g (50 mg/mL) |
| dipotassium phosphate | 1.56 g (90 mM) | 1.56 g (90 mM) |
| methyl parahydroxybenzoate | 0.25 g (2.5 mg/mL) | 0.25 g (2.5 mg/mL) |
| xanthan gum | - | 0.8 g (8.0 mg/mL) |
| sodium alginate | - | 0.8 g (8.0 mg/mL) |
| purified water | 100 mL in total | 100 mL in total |
| Formulation amount (Content) | Example 26 | |
| | Solution A | Solution B |
| Compound 1 hydrochloride | 8.0 g (80 mg/mL) | |
| hypromellose | 2.0 g (20 mg/mL) | - |
| propylene glycol | 5.0 g (50 mg/mL) | 5.0 g (50 mg/mL) |
| dipotassium | 1.56 g (90 mM) | 1.56 g (90 mM) |
| phosphate | | |
| methyl parahydroxybenzoate | 0.25 g (2.5 mg/mL) | 0.25 g (2.5 mg/mL) |
| xanthan gum | - | 0.8 g (8.0 mg/mL) |
| sodium alginate | - | 1.2 g (12 mg/mL) |
| purified water | 100 mL in total | 100 mL in total |

(continued)

| Formulation amount (Content) | Example 26 | |
|---|---|---|
| | Solution A | Solution B |
| Formulation amount (Content) | Example 27 | |
| | Solution A | Solution B |
| Compound 1 hydrochloride | 8.0 g (80 mg/mL) | |
| hypromellose | 2.0 g (20 mg/mL) | - |
| propylene glycol | 5.0 g (50 mg/mL) | 5.0 g (50 mg/mL) |
| dipotassium phosphate | 1.56 g (90 mM) | 1.56 g (90 mM) |
| methyl parahydroxybenzoate | 0.25 g (2.5 mg/mL) | 0.25 g (2.5 mg/mL) |
| carboxy vinyl polymer | | 1.2 g (12 mg/mL) |
| purified water | 100 mL in total | 100 mL in total |

The formulation amount and content of each ingredient in the preparations of the above Examples are shown in Table below.

| Formulation amount (Content) | Example | | |
|---|---|---|---|
| | 24 | 25 | 26 |
| Compound 1 hydrochloride | 8.0 g (40 mg/mL) | 8.0 g (40 mg/mL) | 8.0 g (40 mg/mL) |
| hypromellose | 2.0 g (10 mg/mL) | 2.0 g (10 mg/mL) | 2.0 g (10 mg/mL) |
| propylene glycol | 10.0 g (50 mg/mL) | 10.0 g (50 mg/mL) | 10.0 g (50 mg/mL) |
| dipotassium phosphate | 3.12 g (90 mM) | 3.12 g (90 mM) | 3.12 g (90 mM) |
| methyl parahydroxybenzoate | 0.5 g (2.5 mg/mL) | 0.5 g (2.5 mg/mL) | 0.5 g (2.5 mg/mL) |
| xanthan gum | 0.8 g (4.0 mg/mL) | 0.8 g (4.0 mg/mL) | 0.8 g (4.0 mg/mL) |
| sodium alginate | 0.4 g (2.0 mg/mL) | 0.8 g (4.0 mg/mL) | 1.2 g (6.0 mg/mL) |
| purified water | 200 mL in total | 200 mL in total | 200 mL in total |
| Formulation amount (Content) | Example | | |
| | 27 | | |
| Compound 1 hydrochloride | 8.0 g (40 mg/mL) | | |
| hypromellose | 4.0 g (20 mg/mL) | | |
| propylene glycol | 10.0 g (50 mg/mL) | | |
| dipotassium phosphate | 3.12 g (90 mM) | | |
| methyl parahydroxybenzoate | 0.5 g (2.5 mg/mL) | | |
| carboxy vinyl polymer | 1.2 g (6.0 mg/mL) | | |
| purified water | 200 mL in total | | |

Examples 28-30

According to the following procedure, the preparations of Examples 28-30 were prepared from Compound 1 hydrochloride, an additive and a solvent in the amounts shown in Table below.

(1) Preparation of Solution A

Hypromellose was added to purified water and dissolved. Dipotassium phosphate and erythritol were added thereto and dissolved. Propylene glycol and methyl parahydroxybenzoate were weighed, methyl parahydroxybenzoate was dissolved in propylene glycol in another container, and then the solution was added to purified water containing dipotassium phosphate and erythritol. To the solution was then added Compound 1 hydrochloride, and the mixture was dispersed with Precision Dispersion/Emulsification machine: CLEARMIX CLM-0.8S (M Technique Co., Ltd.) at 8000 rpm for 10 minutes to prepare Solution A.

(2) Preparation of Solution B

Dipotassium phosphate and erythritol were added to purified water and dissolved. Propylene glycol and methyl parahydroxybenzoate were then weighed, methyl parahydroxybenzoate was dissolved in propylene glycol in another container, and then the solution was added to purified water containing dipotassium phosphate and erythritol. The solution was then warmed to 70°C, and xanthan gum was added thereto and dissolved. The solution was then cooled to room temperature to prepare Solution B.

(3) Preparation of Examples 28-30

Solution A and Solution B were mixed in a mass ratio of 1:1 to prepare the preparations of Examples 28-30.

| Formulation amount (Content) | Example 28 | |
|---|---|---|
| | Solution A | Solution B |
| Compound 1 hydrochloride | 8.0 g (80 mg/mL) | - |
| hypromellose | 2.0 g (20 mg/mL) | - |
| propylene glycol | 5.0 g (50 mg/mL) | 5.0 g (50 mg/mL) |
| dipotassium phosphate | 1.56 g (90 mM) | 1.56 g (90 mM) |
| methyl parahydroxybenzoate | 0.25 g (2.5 mg/mL) | 0.25 g (2.5 mg/mL) |
| xanthan gum | - | 0.8 g (8.0 mg/mL) |
| erythritol | 5.0 g (50 mg/mL) | 5.0 g (50 mg/mL) |
| purified water | 100 mL in total | 100 mL in total |
| Formulation amount (Content) | Example 29 | |
| | Solution A | Solution B |
| Compound 1 hydrochloride | 8.0 g (80 mg/mL) | - |
| hypromellose | 2.0 g (20 mg/mL) | - |
| propylene glycol | 5.0 g (50 mg/mL) | 5.0 g (50 mg/mL) |
| dipotassium phosphate | 1.56 g (90 mM) | 1.56 g (90 mM) |
| methyl parahydroxybenzoate | 0.25 g (2.5 mg/mL) | 0.25 g (2.5 mg/mL) |
| xanthan gum | - | 0.8 g (8.0 mg/mL) |
| erythritol | 10.0 g (100 mg/mL) | 10.0 g (100 mg/mL) |
| purified water | 100 mL in total | 100 mL in total |
| Formulation amount (Content) | Example 30 | |
| | Solution A | Solution B |
| Compound 1 hydrochloride | 8.0 g (80 mg/mL) | - |
| hypromellose | 2.0 g (20 mg/mL) | - |
| propylene glycol | 5.0 g (50 mg/mL) | 5.0 g (50 mg/mL) |

(continued)

| Formulation amount (Content) | Example 30 | |
|---|---|---|
| | Solution A | Solution B |
| dipotassium phosphate | 1.56 g (90 mM) | 1.56 g (90 mM) |
| methyl parahydroxybenzoate | 0.25 g (2.5 mg/mL) | 0.25 g (2.5 mg/mL) |
| xanthan gum | - | 0.8 g (8.0 mg/mL) |
| erythritol | 15.0 g (150 mg/mL) | 15.0 g (150 mg/mL) |
| purified water | 100 mL in total | 100 mL in total |

The formulation amount and content of each ingredient in the preparations of the above Examples are shown in Table below.

| Formulation amount (Content) | Example | | |
|---|---|---|---|
| | 28 | 29 | 30 |
| Compound 1 hydrochloride | 8.0 g (40 mg/mL) | 8.0 g (40 mg/mL) | 8.0 g (40 mg/mL) |
| hypromellose | 2.0 g (10 mg/mL) | 2.0 g (10 mg/mL) | 2.0 g (10 mg/mL) |
| propylene glycol | 10.0 g (50 mg/mL) | 10.0 g (50 mg/mL) | 10.0 g (50 mg/mL) |
| dipotassium phosphate | 3.12 g (90 mM) | 3.12 g (90 mM) | 3.12 g (90 mM) |
| methyl parahydroxybenzoate | 0.5 g (2.5 mg/mL) | 0.5 g (2.5 mg/mL) | 0.5 g (2.5 mg/mL) |
| xanthan gum | 0.8 g (4.0 mg/mL) | 0.8 g (4.0 mg/mL) | 0.8 g (4.0 mg/mL) |
| erythritol | 10.0 g (50 mg/mL) | 20.0 g (100 mg/mL) | 30.0 g (150 mg/mL) |
| purified water | 200 mL in total | 200 mL in total | 200 mL in total |

Examples 31 and 32

According to the following procedure, the preparations of Examples 31 and 32 were prepared from Compound 1 hydrochloride, an additive and a solvent in the amounts shown in Table below.

(1) Preparation of Solution A

Hypromellose was added to purified water and dissolved. Sodium chloride and erythritol were then added and dissolved. Propylene glycol and methyl parahydroxybenzoate were then weighed, methyl parahydroxybenzoate was dissolved in propylene glycol in another container, and then the solution was added to purified water containing sodium chloride and erythritol. To the solution was then added Compound 1 hydrochloride, and the mixture was dispersed with Precision Dispersion/Emulsification machine: CLEARMIX CLM-0.8S (M Technique Co., Ltd.) at 8000 rpm for 10 minutes to prepare Solution A.

(2) Preparation of Solution B

Sodium chloride and erythritol were added to purified water and dissolved. Propylene glycol and methyl parahydroxybenzoate were weighed, methyl parahydroxybenzoate was dissolved in propylene glycol in another container, and then the solution was added to purified water containing sodium chloride and erythritol. The solution was then warmed to 70°C, and xanthan gum was added thereto and dissolved. The solution was then cooled to room temperature to prepare Solution B.

(3) Preparation of Examples 31 and 32

Solution A and Solution B were mixed in a mass ratio of 1:1 to prepare the preparations of Examples 31 and 32.

| Formulation amount (Content) | Example 31 | |
|---|---|---|
| | Solution A | Solution B |
| Compound 1 hydrochloride | 8.0 g (80 mg/mL) | - |
| hypromellose | 2.0 g (20 mg/mL) | - |
| propylene glycol | 5.0 g (50 mg/mL) | 5.0 g (50 mg/mL) |
| sodium chloride | 1.0 g (10 mg/mL) | 1.0 g (10 mg/mL) |
| methyl parahydroxybenzoate | 0.2 g (2.0 mg/mL) | 0.2 g (2.0 mg/mL) |
| xanthan gum | - | 1.2 g (12.0 mg/mL) |
| erythritol | 20 g (200 mg/mL) | 20 g (200 mg/mL) |
| purified water | 100 mL in total | 100 mL in total |
| Formulation amount (Content) | Example 32 | |
| | Solution A | Solution B |
| Compound 1 hydrochloride | 8.0 g (80 mg/mL) | - |
| hypromellose | 2.0 g (20 mg/mL) | - |
| propylene glycol | 5.0 g (50 mg/mL) | 5.0 g (50 mg/mL) |
| sodium chloride | 1.0 g (10 mg/mL) | 1.0 g (10 mg/mL) |
| methyl parahydroxybenzoate | 0.2 g (2.0 mg/mL) | 0.2 g (2.0 mg/mL) |
| xanthan gum | - | 1.0 g (10.0 mg/mL) |
| erythritol | 20 g (200 mg/mL) | 20 g (200 mg/mL) |
| purified water | 100 mL in total | 100 mL in total |

The formulation amount and content of each ingredient in the preparations of the above Examples are shown in Table below.

| Formulation amount (Content) | Example | |
|---|---|---|
| | 31 | 32 |
| Compound 1 hydrochloride | 8.0 g (40 mg/mL) | 8.0 g (40 mg/mL) |
| hypromellose | 2.0 g (10 mg/mL) | 2.0 g (10 mg/mL) |
| propylene glycol | 10.0 g (50 mg/mL) | 10.0 g (50 mg/mL) |
| sodium chloride | 2.0 g (10 mg/mL) | 2.0 g (10 mg/mL) |
| methyl parahydroxybenzoate | 0.4 g (2.0 mg/mL) | 0.4 g (2.0 mg/mL) |
| xanthan gum | 1.2 g (6.0 mg/mL) | 1.0 g (5.0 mg/mL) |
| erythritol | 40.0 g (200 mg/mL) | 40.0 g (200 mg/mL) |
| purified water | 200 mL in total | 200 mL in total |

Examples 33 and 34

According to the following procedure, the preparations of Examples 33 and 34 were prepared from Compound 1 hydrochloride, an additive and a solvent in the amounts shown in Table below.

(1) Preparation of Solution A

Hypromellose was added to purified water and dissolved. Sodium benzoate, propylene glycol and erythritol

were added thereto and dissolved. To the solution was then added Compound 1 hydrochloride, and the mixture was dispersed with Precision Dispersion/Emulsification machine: CLEARMIX CLM-0.8S (M Technique Co., Ltd.) at 8000 rpm for 10 minutes to prepare Solution A.

(2) Preparation of Solution B

Sodium benzoate, propylene glycol and erythritol were added to purified water and dissolved. The solution was then warmed to 70°C, and xanthan gum was added thereto and dissolved. The solution was then cooled to room temperature to prepare Solution B.

(3) Preparation of Examples 33 and 34

Solution A and Solution B were mixed in a mass ratio of 1:1 to prepare the preparations of Examples 33 and 34.

| Formulation amount (Content) | Example 33 | |
|---|---|---|
| | Solution A | Solution B |
| Compound 1 hydrochloride | 8.0 g (80 mg/mL) | - |
| hypromellose | 2.0 g (20 mg/mL) | - |
| propylene glycol | 5.0 g (50 mg/mL) | 5.0 g (50 mg/mL) |
| sodium benzoate | 0.2 g (2.0 mg/mL) | 0.2 g (2.0 mg/mL) |
| xanthan gum | - | 0.8 g (8.0 mg/mL) |
| erythritol | 20 g (200 mg/mL) | 20 g (200 mg/mL) |
| purified water | 100 mL in total | 100 mL in total |
| Formulation amount (Content) | Example 34 | |
| | Solution A | Solution B |
| Compound 1 hydrochloride | 8.0 g (80 mg/mL) | - |
| hypromellose | 3.0 g (30 mg/mL) | - |
| propylene glycol | 5.0 g (50 mg/mL) | 5.0 g (50 mg/mL) |
| sodium benzoate | 0.2 g (2.0 mg/mL) | 0.2 g (2.0 mg/mL) |
| xanthan gum | - | 0.8 g (8.0 mg/mL) |
| erythritol | 20 g (200 mg/mL) | 20 g (200 mg/mL) |
| purified water | 100 mL in total | 100 mL in total |

The formulation amount and content of each ingredient in the preparations of the above Examples are shown in Table below.

| Formulation amount (Content) | Example | |
|---|---|---|
| | 33 | 34 |
| Compound 1 hydrochloride | 8.0 g (40 mg/mL) | 8.0 g (40 mg/mL) |
| hypromellose | 2.0 g (10 mg/mL) | 3.0 g (15 mg/mL) |
| propylene glycol | 10.0 g (50 mg/mL) | 10.0 g (50 mg/mL) |
| sodium benzoate | 0.4 g (2.0 mg/mL) | 0.4 g (2.0 mg/mL) |
| xanthan gum | 0.8 g (4.0 mg/mL) | 0.8 g (4.0 mg/mL) |
| erythritol | 40.0 g (200 mg/mL) | 40.0 g (200 mg/mL) |
| purified water | 200 mL in total | 200 mL in total |

Example 35

According to the following procedure, the preparation of Example 35 was prepared from Compound 1 hydrochloride, an additive and a solvent in the amounts shown in Table below.

Sodium chloride, sodium benzoate and sorbitol were added to purified water and dissolved. The solution was then warmed to 70°C, and xanthan gum was added thereto and dissolved. The solution was then cooled to room temperature, To the solution was then added Compound 1 hydrochloride, and the mixture was dispersed with Precision Dispersion/Emulsification machine: CLEARMIX CLM-0.8S (M Technique Co., Ltd.) at 15000 rpm for 10 minutes to prepare the preparation of Example 35.

| Formulation amount (Content) | Example |
|---|---|
| | 35 |
| Compound 1 hydrochloride | 8.0 g (40 mg/mL) |
| sodium chloride | 1.0 g (5 mg/mL) |
| sodium benzoate | 0.4 g (2 mg/mL) |
| sorbitol | 60.0 g (300 mg/mL) |
| xanthan gum | 0.4 g (2 mg/mL) |
| purified water | 200 mL in total |

Comparative Examples 1-7

According to the procedure of Examples 1-5, the preparations of Comparative Examples 1-7 were prepared.

| Formulation amount (Content) | Comparative Example | | |
|---|---|---|---|
| | 1 | 2 | 3 |
| Compound 1 hydrochloride | 8.0 g (40 mg/mL) | 8.0 g (40 mg/mL) | 8.0 g (40 mg/mL) |
| HCO-60 | 0.2 g (1 mg/mL) | - | - |
| polyglycerol fatty acid ester | - | 0.2 g (1 mg/mL) | - |
| polyvinylpyrrolidone | - | - | 0.2 g (1 mg/mL) |
| dipotassium phosphate | 3.12 g (90 mM) | 3.12 g (90 mM) | 3.12 g (90 mM) |
| methyl parahydroxybenzoate | 0.1 g (0.5 mg/mL) | 0.1 g (0.5 mg/mL) | 0.1 g (0.5 mg/mL) |
| purified water | 200 mL in total | 200 mL in total | 200 mL in total |
| Formulation amount (Content) | Comparative Example | | |
| | 4 | 5 | 6 |
| Compound 1 hydrochloride | 8.0 g (40 mg/mL) | 8.0 g (40 mg/mL) | 8.0 g (40 mg/mL) |
| Poloxamer 188 | 0.2 g (1 mg/mL) | - | - |
| Polysorbate 80 | - | 0.2 g (1 mg/mL) | - |
| Polyoxyl 40 Stearate | - | - | 0.2 g (1 mg/mL) |
| dipotassium phosphate | 3.12 g (90 mM) | 3.12 g (90 mM) | 3.12 g (90 mM) |
| methyl parahydroxybenzoate | 0.1 g (0.5 mg/mL) | 0.1 g (0.5 mg/mL) | 0.1 g (0.5 mg/mL) |
| purified water | 200 mL in total | 200 mL in total | 200 mL in total |
| Formulation amount (Content) | Comparative Example | | |
| | 7 | | |
| Compound 1 hydrochloride | 8.0 g (40 mg/mL) | | |

(continued)

| Formulation amount (Content) | Comparative Example |
|---|---|
| | 7 |
| sodium lauryl sulfate | 0.2 g (1 mg/mL) |
| dipotassium phosphate | 3.12 g (90 mM) |
| methyl parahydroxybenzoate | 0.1 g (0.5 mg/mL) |
| purified water | 200 mL in total |

Comparative Examples 8 and 9

According to the procedure of Example 6, the preparations of Comparative Examples 8 and 9 were prepared.

| Formulation amount (Content) | Comparative Example | |
|---|---|---|
| | 8 | 9 |
| Compound 1 hydrochloride | 8.0 g (40 mg/mL) | 8.0 g (40 mg/mL) |
| Poloxamer 188 | 0.2 g (1 mg/mL) | - |
| Polysorbate 80 | - | 0.2 g (1 mg/mL) |
| Propylene glycol | 10.0 g (50 mg/mL) | 10.0 g (50 mg/mL) |
| Dipotassium phosphate | 3.12 g (90 mM) | 3.12 g (90 mM) |
| Methyl parahydroxybenzoate | 0.5 g (2.5 mg/mL) | 0.5 g (2.5 mg/mL) |
| Purified water | 200 mL in total | 200 mL in total |

Comparative Examples 10-13

According to the procedure of Examples 7-10, the preparations of Comparative Examples 10-13 were prepared.

| Formulation amount (Content) | Comparative Example | | |
|---|---|---|---|
| | 10 | 11 | 12 |
| Compound 1 hydrochloride | 8.0 g (40 mg/mL) | 8.0 g (40 mg/mL) | 8.0 g (40 mg/mL) |
| Hypromellose | 0.2 g (1 mg/mL) | 0.2 g (1 mg/mL) | - |
| HCO-60 | 0.2 g (1 mg/mL) | 0.4 g (2 mg/mL) | 0.2 g (1 mg/mL) |
| Sucrose laurate | - | - | 0.2 g (1 mg/mL) |
| Dipotassium phosphate | 3.12 g (90 mM) | 3.12 g (90 mM) | 3.12 g (90 mM) |
| Methyl parahydroxybenzoate | 0.1 g (0.5 mg/mL) | 0.1 g (0.5 mg/mL) | 0.1 g (0.5 mg/mL) |
| Purified water | 200 mL in total | 200 mL in total | 200 mL in total |

| Formulation amount (Content) | Comparative Example |
|---|---|
| | 13 |
| Compound 1 hydrochloride | 8.0 g (40 mg/mL) |
| Hypromellose | 0.2 g (1 mg/mL) |
| HCO-60 | 0.8 g (4 mg/mL) |
| Dipotassium phosphate | 3.12 g (90 mM) |
| Methyl parahydroxybenzoate | 0.1 g (0.5 mg/mL) |

(continued)

| Formulation amount (Content) | Comparative Example |
|---|---|
| | 13 |
| Purified water | 200 mL in total |

Comparative Examples 14-16

According to the procedure of Example 13, the preparations of Comparative Examples 14-16 were prepared.

| Formulation amount (Content) | Example | | |
|---|---|---|---|
| | 14 | 15 | 16 |
| Compound 1 hydrochloride | 8.0 g (40 mg/mL) | 8.0 g (40 mg/mL) | 8.0 g (40 mg/mL) |
| Poloxamer 188 | 2.0 g (10 mg/mL) | - | - |
| Polysorbate 80 | - | 2.0 g (10 mg/mL) | - |
| Polyvinyl alcohol | - | - | 2.0 g (10 mg/mL) |
| Propylene glycol | 10.0 g (50 mg/mL) | 10.0 g (50 mg/mL) | 10.0 g (50 mg/mL) |
| Dipotassium phosphate | 3.12 g (90 mM) | 3.12 g (90 mM) | 3.12 g (90 mM) |
| Methyl parahydroxybenzoate | 0.5 g (2.5 mg/mL) | 0.5 g (2.5 mg/mL) | 0.5 g (2.5 mg/mL) |
| Xanthan gum | 0.8 g (4 mg/mL) | 0.8 g (4 mg/mL) | 0.8 g (4 mg/mL) |
| Purified water | 200 mL in total | 200 mL in total | 200 mL in total |

Comparative Examples 17 and 18

According to the procedure of Examples 14 and 15, the preparations of Comparative Examples 17 and 18 were prepared.

| Formulation amount (Content) | Comparative Example | |
|---|---|---|
| | 17 | 18 |
| Compound 1 hydrochloride | 4.0 g (20 mg/mL) | 4.0 g (20 mg/mL) |
| Poloxamer 188 | 0.2 g (1.0 mg/mL) | 1.0 g (5.0 mg/mL) |
| Monopotassium phosphate | 0.82 g (30 mM) | 0.82 g (30 mM) |
| Dipotassium phosphate | 2.44 g (70 mM) | 2.44 g (70 mM) |
| Methyl parahydroxybenzoate | 0.4 g (2.0 mg/mL) | 0.4 g (2.0 mg/mL) |
| Xanthan gum | 0.8 g (4.0 mg/mL) | 0.8 g (4.0 mg/mL) |
| Purified water | 200 mL in total | 200 mL in total |

Test Example 1 (Manufacturability Evaluation)

The preparations of Examples 1-5 and Comparative Examples 1-7 were prepared and then allowed to stand at room temperature for 1 hour. After standing, the preparation in which the suspended particle was not emerged on the solution surface and was dispersed or precipitated in the solution was determined as being good manufacturability (A), and the preparation in which the suspended particle was emerged on the solution surface was determined as being poor manufacturability (B).

| | Manufacturability Evaluation |
|---|---|
| Example 1 | A |
| Example 2 | A |
| Example 3 | A |
| Example 4 | A |
| Example 5 | A |
| Comparative Example 1 | A |
| Comparative Example 2 | A |
| Comparative Example 3 | B |
| Comparative Example 4 | A |
| Comparative Example 5 | A |
| Comparative Example 6 | A |
| Comparative Example 7 | B |

Test Example 2 (Measurement of Circularity)

According to (II) Measurement method of circularity herein, the circularity of each suspended particle in the preparations of Examples and Comparative Examples shown in Table below was measured.

| | Circularity |
|---|---|
| Example 1 | 0.931 |
| Example 2 | 0.942 |
| Example 3 | 0.900 |
| Example 4 | 0.931 |
| Example 5 | 0.930 |
| Comparative Example 1 | 0.961 |
| Comparative Example 2 | 0.951 |
| Comparative Example 4 | 0.956 |
| Comparative Example 5 | 0.958 |
| Comparative Example 6 | 0.960 |
| | Circularity |
| Example 6 | 0.927 |
| Comparative Example 8 | 0.966 |
| Comparative Example 9 | 0.948 |
| | Circularity |
| Example 7 | 0.932 |
| Example 8 | 0.939 |
| Example 9 | 0.943 |
| Example 10 | 0.945 |
| Example 11 | 0.926 |
| Example 12 | 0.928 |

(continued)

| | Circularity |
|---|---|
| Comparative Example 10 | 0.954 |
| Comparative Example 11 | 0.958 |
| Comparative Example 12 | 0.966 |
| Comparative Example 13 | 0.963 |
| | Circularity |
| Example 13 | 0.935 |
| Comparative Example 14 | 0.967 |
| Comparative Example 15 | 0.967 |
| Comparative Example 16 | 0.963 |
| | Circularity |
| Example 14 | 0.919 |
| Example 15 | 0.925 |
| Comparative Example 17 | 0.962 |
| Comparative Example 18 | 0.972 |
| | Circularity |
| Example 16 | 0.954 |
| Example 17 | 0.955 |
| Example 18 | 0.954 |
| Example 19 | 0.957 |
| Example 20 | 0.882 |
| Example 21 | 0.918 |
| Example 22 | 0.936 |
| Example 23 | 0.939 |
| Example 24 | 0.941 |
| Example 25 | 0.940 |
| Example 26 | 0.940 |
| Example 27 | 0.865 |
| Example 28 | 0.938 |
| Example 29 | 0.936 |
| Example 30 | 0.937 |
| Example 31 | 0.938 |
| Example 32 | 0.938 |
| Example 33 | 0.933 |
| Example 34 | 0.932 |
| Example 35 | 0.934 |

Test Example 3 (Measurement of median diameter)

According to (III) Measurement method of median diameter herein, the median diameter of each suspended

particle of Compound 1 hydrochloride in the preparations of Examples and Comparative Examples shown in Table below was measured.

| | Median Diameter (μm) |
|---|---|
| Example 1 | 5.17 |
| Example 2 | 3.06 |
| Example 3 | 12.44 |
| Example 4 | 3.96 |
| Example 5 | 4.71 |
| Comparative Example 1 | 2.62 |
| Comparative Example 2 | 3.98 |
| Comparative Example 4 | 12.93 |
| Comparative Example 5 | 3.79 |
| Comparative Example 6 | 4.19 |
| | Median Diameter (μm) |
| Example 6 | 7.53 |
| Comparative Example 8 | 2.56 |
| Comparative Example 9 | 6.50 |
| | Median Diameter (μm) |
| Example 7 | 3.95 |
| Example 8 | 3.25 |
| Example 9 | 3.39 |
| Example 10 | 4.44 |
| Example 11 | 6.01 |
| Example 12 | 6.07 |
| Comparative Example 10 | 2.06 |
| Comparative Example 11 | 2.05 |
| Comparative Example 12 | 2.31 |
| Comparative Example 13 | 2.23 |
| | Median Diameter (μm) |
| Example 13 | 2.60 |
| Comparative Example 14 | 2.70 |
| Comparative Example 15 | 3.52 |
| Comparative Example 16 | 2.50 |
| | Median Diameter (μm) |
| Example 14 | 3.34 |
| Example 15 | 3.30 |
| Comparative Example 17 | 3.00 |
| Comparative Example 18 | 2.26 |
| | Median Diameter (μm) |
| Example 16 | 3.11 |

(continued)

| | Median Diameter (μm) |
|---|---|
| Example 17 | 4.25 |
| Example 18 | 3.56 |
| Example 19 | 4.13 |
| Example 20 | 4.64 |
| Example 21 | 3.90 |
| Example 22 | 2.89 |
| Example 23 | 2.62 |
| Example 24 | 3.22 |
| Example 25 | 3.05 |
| Example 26 | 2.96 |
| Example 27 | 14.67 |
| Example 28 | 2.73 |
| Example 29 | 3.02 |
| Example 30 | 3.09 |
| Example 31 | 2.61 |
| Example 32 | 2.57 |
| Example 33 | 3.12 |
| Example 34 | 3.73 |
| Example 35 | 2.95 |

Test Example 4 (Measurement of Rsp)

According to (IV) Measurement method of Rsp herein, the Rsp value of the preparations of Examples and Comparative Examples shown in Table below was measured.

| | Rsp |
|---|---|
| Example 1 | 0.47 |
| Example 2 | 0.52 |
| Comparative Example 1 | 0.11 |
| Comparative Example 2 | 0.01 |
| Comparative Example 4 | 0.07 |
| Comparative Example 5 | 0.01 |
| Comparative Example 6 | 0.10 |
| | Rsp |
| Example 6 | 0.28 |
| Comparative Example 8 | 0.14 |
| Comparative Example 9 | 0.02 |
| | Rsp |
| Example 8 | 0.26 |

(continued)

| | Rsp |
|---|---|
| Example 9 | 0.36 |
| Example 10 | 0.44 |
| Comparative Example 10 | 0.24 |
| Comparative Example 11 | 0.21 |
| Comparative Example 12 | 0.23 |
| Comparative Example 13 | 0.20 |
| | Rsp |
| Example 13 | 0.34 |
| Comparative Example 14 | 0.22 |
| Comparative Example 15 | 0.19 |
| Comparative Example 16 | 0.20 |

Test Example 5(Redispersibility evaluation)

According to (I) Evaluation method of redispersibility as used herein, the preparations of Examples and Comparative Examples shown in Table below were stored with standing at 25°C or 40°C for 1 month, and then the redispersibility of the preparations was evaluated.

| | Redispersibility in product stored at 25°C | Redispersibility in product stored at 40°C |
|---|---|---|
| Example 1 | A | A |
| Example 2 | A | A |
| Example 3 | A | A |
| Example 4 | A | A |
| Example 5 | A | A |
| Comparative Example 1 | C | C |
| Comparative Example 2 | C | C |
| Comparative Example 4 | C | C |
| Comparative Example 5 | C | C |
| Comparative Example 6 | C | C |
| | Redispersibility in product stored at 25°C | Redispersibility in product stored at 40°C |
| Example 6 | A | A |
| Comparative Example 8 | C | C |
| Comparative Example 9 | C | B |
| | Redispersibility in product stored at 25°C | Redispersibility in product stored at 40°C |
| Example 7 | B | B |
| Example 8 | B | A |
| Example 9 | B | A |
| Example 10 | B | A |
| Example 11 | A | A |
| Example 12 | A | A |

(continued)

| | Redispersibility in product stored at 25°C | Redispersibility in product stored at 40°C |
|---|---|---|
| Comparative Example 10 | B | C |
| Comparative Example 11 | B | C |
| Comparative Example 12 | C | C |
| Comparative Example 13 | B | C |

According to (I) Evaluation method of redispersibility as used herein, the preparations of Examples and Comparative Examples shown in Table below were stored with standing at 25°C or 40°C for 3 months, and then the redispersibility of the preparations was evaluated.

| | Redispersibility in product stored at 25°C | Redispersibility in product stored at 40°C |
|---|---|---|
| Example 13 | A | A |
| Comparative Example 14 | A | C |
| Comparative Example 15 | A | C |
| Comparative Example 16 | A | C |

According to (I) Evaluation method of redispersibility as used herein, the preparations of Examples and Comparative Examples shown in Table below were stored with standing at 25°C 24 months, and then the redispersibility of the preparations was evaluated.

| | Redispersibility in product stored at 25°C |
|---|---|
| Example 14 | A |
| Example 15 | A |
| Comparative Example 17 | C |
| Comparative Example 18 | C |

According to (I) Evaluation method of redispersibility as used herein, the preparations of Examples and Comparative Examples shown in Table below were stored with standing at 25°C for 6 months, and then the redispersibility of the preparations was evaluated.

| | Redispersibility in product stored at 25°C |
|---|---|
| Example 16 | A |
| Example 17 | A |
| Example 18 | B |
| Example 19 | B |
| Example 20 | A |
| Example 21 | A |
| Example 22 | A |
| Example 23 | A |

According to (I) Evaluation method of redispersibility as used herein, the preparations of Examples and Comparative Examples shown in Table below were stored with standing at 25°C for 18 months, and then the redispersibility of the preparations was evaluated.

| | Redispersibility in product stored at 25°C |
|---|---|
| Example 24 | A |
| Example 25 | A |
| Example 26 | A |

In Test Examples 2 and 5, the results of the aqueous suspension preparations comprising no thickening agent in Examples 1-12 and Comparative Examples 1-13 were compared. As a result, it was shown that the preparations of Examples 1-12 wherein the circularity of the suspended particle is 0.945 or less had good redispersibility, and the preparations of Comparative Examples 1-13 wherein the circularity of the suspended particle is more than 0.945 had poor redispersibility. Also, the results of the aqueous suspension preparations comprising a thickening agent in Examples 13-26 and Comparative Examples 14-18 were compared. As a result, it was shown that the preparations of Examples 13-26 wherein the circularity of the suspended particle is 0.960 or less had good redispersibility, and the preparations of Comparative Examples 14-18 wherein the circularity of the suspended particle is more than 0.960 had poor redispersibility

In Test Examples 3 and 5, it was shown that all the preparations of Examples 1-35 in which the suspended particle of Compound 1 hydrochloride had a median diameter of 0.1 - about 30 μm had good redispersibility.

In Test Examples 4 and 5, it was shown that the preparations of Examples 1, 2, 6, 8, 9, 10 and 13 (wherein the Rsp value of Compound 1 hydrochloride suspended in the preparation which was measured with pulse NMR is 0.25 or more, wherein said measured concentration was 40 mg/mL) had good redispersibility. Whereas, the preparations of Comparative Examples 1, 2, 4, 5, 6, 8, 9, 10, 11, 12, 13, 14, 15, and 16 wherein the Rsp value was less than 0.25 had poor redispersibility.

INDUSTRIAL APPLICABILITY

The present invention can provide an aqueous suspension preparation with an excellent redispersibility of Compound 1 or salt thereof. In the aqueous suspension preparation of the present invention, the precipitate formed by standing for a long time can easily return to the initial homogeneous suspended state. Thus, it is expected that the aqueous suspension preparation of the present invention can reduce the variation in a dosage for each user or each use.

**Claims**

1. An aqueous suspension preparation comprising (3aR,4S,7R,7aS)-2-{(1R,2R)-2-[4-(1,2-benzisothiazol-3-yl)piperazin-1-ylmethyl]cyclohexylmethyl}hexahydro-4,7-methano-2H-isoindole-1,3-dione (Compound 1), a pharmaceutically acceptable acid addition salt thereof, or a mixture thereof as an active ingredient, which has the following feature (I) or (II):

   (I) the circularity of the suspended particle is about 0.960 or less when the preparation comprises a thickening agent,
   (II) the circularity of the suspended particle is about 0.945 or less when the preparation comprises no thickening agent.

2. An aqueous suspension preparation comprising (3aR,4S,7R,7aS)-2-{(1R,2R)-2-[4-(1,2-benzisothiazol-3-yl)piperazin-1-ylmethyl]cyclohexylmethyl}hexahydro-4,7-methano-2H-isoindole-1,3-dione (Compound 1), a pharmaceutically acceptable acid addition salt thereof, or a mixture thereof, wherein the circularity of the suspended particle is about 0.945 or less.

3. The preparation according to claim 1 or 2, wherein the median diameter of the suspended particle of Compound 1, a pharmaceutically acceptable acid addition salt thereof, or a mixture thereof is about 0.1 - about 30 μm.

4. The preparation according to any one of claims 1 to 3, wherein the Rsp value of Compound 1, a pharmaceutically acceptable acid addition salt thereof, or a mixture thereof suspended in the preparation which is measured with pulse NMR is about 0.25 or more, wherein said measured concentration is diluted to 40 mg/mL (in terms of Compound 1 hydrochloride).

**5.** The preparation according to any one of claims 1 to 4, wherein the content of Compound 1, a pharmaceutically acceptable acid addition salt thereof, or a mixture thereof is about 0.1 - about 400 mg/mL in terms of Compound 1 hydrochloride.

**6.** The preparation according to any one of claims 1 to 5 which further comprises a dispersant.

**7.** The preparation according to claim 6, wherein the dispersant is at least one ingredient selected from the group consisting of cellulose derivatives, sucrose fatty acid esters and polyvinyl alcohol.

**8.** The preparation according to claim 6 or 7, wherein the content of the dispersant is about 0.1 - about 20 mg/mL.

**9.** The preparation according to any one of claims 1 to 8, which comprises a thickening agent.

**10.** The preparation according to claim 9, wherein the thickening agent is at least one ingredient selected from polysaccharides.

**11.** The preparation according to claim 9 or 10, wherein the content of the thickening agent is about 0.5 - about 20 mg/mL.

**12.** The preparation according to any one of claims 1 to 11, which further comprises a buffering agent.

**13.** The preparation according to claim 12, wherein the buffering agent is at least one ingredient selected from the group consisting of sodium salt, potassium salt, and hydrate thereof.

**14.** The preparation according to claim 12 or 13, wherein the buffering agent is contained in an amount of about 0.01 - about 15 mg relative to 1 mg of Compound 1, a pharmaceutically acceptable acid addition salt thereof, or a mixture thereof in terms of Compound 1 hydrochloride.

**15.** The preparation according to any one of claims 1 to 14 which further comprises a preservative.

**16.** The preparation according to claim 15, wherein the preservative is at least one ingredient selected from benzoic acid derivatives.

**17.** The preparation according to claim 15 or 16, wherein the content of the preservative is about 0.1 - about 10 mg/mL.

**18.** The preparation according to any one of claims 1 to 17 which further comprises a stabilizing agent.

**19.** The preparation according to claim 18, wherein the stabilizing agent is at least one ingredient selected from polyalcohols.

**20.** The preparation according to claim 18 or 19, wherein the content of the stabilizing agent is about 1 - about 500 mg/mL.

**21.** The preparation according to any one of claims 1 to 20, wherein the circularity of the suspended particle is about 0.960 or less, comprising the following (1) to (6):

(1) Compound 1, a pharmaceutically acceptable acid addition salt thereof, or a mixture thereof in a content of about 0.1 - about 400 mg/mL in terms of Compound 1 hydrochloride,
(2) a dispersant in an amount of about 0.1 - about 20 mg/mL, which is at least one ingredient selected from the group consisting of cellulose derivatives and sucrose fatty acid esters,
(3) a thickening agent in a content of about 0.5 - about 20 mg/mL, which is at least one ingredient selected from polysaccharides,
(4) a buffering agent in an amount of about 0.01 - about 15 mg relative to 1 mg of Compound 1, a pharmaceutically acceptable acid addition salt thereof, or a mixture thereof in terms of Compound 1 hydrochloride, which is at least one ingredient selected from the group consisting of sodium salt, potassium salt and hydrate thereof,
(5) a preservative in a content of about 0.1 - about 10 mg/mL, which is at least one ingredient selected from benzoic acid derivatives, and
(6) a stabilizing agent in a content of about 1 - about 500 mg/mL, which is at least one ingredient selected from polyalcohols.

**22.** The preparation according to any one of claims 1 to 21, which comprises Compound 1 hydrochloride as Compound 1, a pharmaceutically acceptable acid addition salt thereof, or a mixture thereof.

**23.** The preparation according to any one of claims 1 to 22, wherein the content of Compound 1, a pharmaceutically acceptable acid addition salt thereof, or a mixture thereof is about 12 - about 100 mg/mL in terms of Compound 1 hydrochloride.

**24.** The preparation according to any one of claims 1 to 23, which is an oral solution.

**25.** A medicament for treatment and/or prophylaxis of a psychiatric disease, which comprises the preparation according to any one of claims 1 to 24.

**26.** The medicament according to claim 25, wherein the psychiatric disease is schizophrenia, bipolar disorder, senile dementia, or depression.

**27.** A method for treatment and/or prophylaxis of a psychiatric disease, which comprises administering the preparation according to any one of claims 1 to 24 to a patient in need thereof.

**28.** Use of the preparation according to any one of claims 1 to 24 for the manufacture of a medicament for treatment and/or prophylaxis of a psychiatric disease.

**29.** The preparation according to any one of claims 1 to 24 for use in the treatment and/or prophylaxis of a psychiatric disease.

## INTERNATIONAL SEARCH REPORT

International application No.
PCT/JP2017/031275

**A. CLASSIFICATION OF SUBJECT MATTER**

*A61K31/496*(2006.01)i, *A61K9/10*(2006.01)i, *A61K47/02*(2006.01)i, *A61K47/10*(2006.01)i, *A61K47/14*(2006.01)i, *A61K47/26*(2006.01)i, *A61K47/32*(2006.01)i, *A61K47/36*(2006.01)i, *A61K47/38*(2006.01)i

According to International Patent Classification (IPC) or to both national classification and IPC

**B. FIELDS SEARCHED**

Minimum documentation searched (classification system followed by classification symbols)

A61K31/496, A61K9/10, A61K47/02, A61K47/10, A61K47/14, A61K47/26, A61K47/32, A61K47/36, A61K47/38

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)

CAplus/REGISTRY/MEDLINE/EMBASE/BIOSIS(STN), JSTPlus/JMEDPlus/JST7580(JDreamIII)

**C. DOCUMENTS CONSIDERED TO BE RELEVANT**

| Category* | Citation of document, with indication, where appropriate, of the relevant passages | Relevant to claim No. |
|---|---|---|
| X<br>Y | CN 104606133 A (AVENTIS PHARMA HAINAN CO., LTD.), 13 May 2015 (13.05.2015), paragraphs [0005], [0006], [0009], [0010], [0017]; examples 1 to 4 (Family: none) | 1-17,22-29<br>18-21 |
| X | JP 2013-543481 A (Dainippon Sumitomo Pharma Co., Ltd.), 05 December 2013 (05.12.2013), claims 1 to 4, 14, 29; paragraphs [0091] to [0093], [0098]<br>& US 2012/0091022 A1 & WO 2012/053654 A1<br>claims 1 to 4, 14, 29; paragraphs [0091] to [0093], [0098]<br>& EP 2629775 A1 & CA 2814840 A<br>& CN 103249416 A & KR 10-2013-0139978 A | 1-3,5,6,8-10,12-14,18,22,25-29 |

☒ Further documents are listed in the continuation of Box C. ☐ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| 14 September 2017 (14.09.17) | 03 October 2017 (03.10.17) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

**INTERNATIONAL SEARCH REPORT**

International application No.
PCT/JP2017/031275

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2007-8852 A (Towa Pharmaceutical Co., Ltd.), 18 January 2007 (18.01.2007), claims (Family: none) | 18-21 |
| Y | JP 2001-316265 A (The Nisshin Oil Mills, Ltd.), 13 November 2001 (13.11.2001), claims; paragraph [0008] (Family: none) | 18-21 |
| A | WO 2008/053920 A1 (Kaneka Corp.), 08 May 2008 (08.05.2008), & US 2008/0102131 A1 & EP 2098222 A1 & CN 101534796 A & TW 200836771 A | 1-29 |
| A | JP 2007-176869 A (Meijo University), 12 July 2007 (12.07.2007), (Family: none) | 1-29 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2800953 B
- US 5532372 A
- WO 2005009999 A
- WO 2006134864 A
- WO 2012053654 A